(19) Europäisches Patentamt European Patent Office Office européen des brevets

(11) **EP 4 645 397 A1**

(12) **EUROPEAN PATENT APPLICATION**
published in accordance with Art. 153(4) EPC

(43) Date of publication:
**05.11.2025 Bulletin 2025/45**

(21) Application number: **23911464.8**

(22) Date of filing: **22.11.2023**

(51) International Patent Classification (IPC):
*H01L 33/62* (2010.01)     *A01G 33/00* (2006.01)
*C12M 1/00* (2006.01)     *H01L 33/00* (2010.01)

(52) Cooperative Patent Classification (CPC):
**A01G 33/00; C12M 1/00; H10H 20/80; H10H 20/857**

(86) International application number:
**PCT/JP2023/041913**

(87) International publication number:
**WO 2024/142682 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022212436**

(71) Applicant: **Dai Nippon Printing Co., Ltd.**
**Tokyo 162-8001 (JP)**

(72) Inventors:
• **KAMEKAWA, Naoto**
**Tokyo 162-8001 (JP)**
• **WATANABE, Masachika**
**Tokyo 162-8001 (JP)**

(74) Representative: **Müller-Boré & Partner**
**Patentanwälte PartG mbB**
**Friedenheimer Brücke 21**
**80639 München (DE)**

(54) **LED SHEET, LED SHEET ASSEMBLY, AND CULTURE SYSTEM**

(57) An LED sheet (20) includes a substrate (31) that has a first surface (31a) and a second surface (31b) positioned on a side opposite to the first surface (31a), a metal wiring portion (32) positioned on the first surface (31a) of the substrate (31), a plurality of LED chips (21) mounted on the metal wiring portion (32), a power receiving terminal (61) connected to the metal wiring portion (32) and used for connection with another LED sheet (20), and a power feeding terminal (62) connected to the metal wiring portion (32) and used for connection with another LED sheet (20).

FIG. 3

EP 4 645 397 A1

## Description

Technical Field

The present disclosure relates to an LED sheet, an LED sheet assembly, and a culture system.

Background Art

In recent years, algal biofuel attracts attention as a renewable energy resource. Methods of culturing algae include so-called open pond systems and so-called closed photobioreactor systems. Of these, closed photobioreactor systems can reduce pollution of a culture solution compared to open pond systems. Closed photobioreactor systems allow year-round production indoors, so the closed photoreactor systems excel in production of algae. As a technology related to the culture of algae using such a photobioreactor, for example, a technique for guiding light from a light-emitting diode into a culture tank through an optical waveguide has been disclosed (see, for example, PTL 1).

In recent years, there are growing demands for lighting units that use low-power consumption LEDs as light sources in place of existing fluorescent lights, high-pressure sodium lights, and the like as lighting units. An LED lighting unit for plant and animal growth, in which a plurality of LED chips is arranged on a flexible circuit board to form a surface light source, is also suggested as an example of a lighting unit that uses an LED as a light source (see, for example, PTL 2).

By the way, in algae culture systems or plant and animal growth factories, such LED lighting units can be used in a state where a plurality of lighting units is connected to each other by connecting the circuit boards of the plurality of LED lighting units. In flexible circuits where connection pads contact the terminals of a connector to establish electrical continuity, it is known to insulate the tip portion that is inserted into the connector as a technology for connecting circuit boards (see, for example, PTL 3).

Citation List

Patent Literature

PTL 1: Japanese Unexamined Patent Application Publication No. 2012-183002

PTL 2: Japanese Unexamined Patent Application Publication No. 2013-251230

PTL 3: Japanese Unexamined Patent Application Publication No. 6-006003

The present disclosure provides an LED sheet, an LED sheet assembly, and a culture system that allow easy connection of LED sheets to each other.

Summary of Invention

A first aspect of the present disclosure is an LED sheet. The LED sheet includes a substrate that has a first surface and a second surface positioned on a side opposite to the first surface, a metal wiring portion positioned on the first surface of the substrate, a plurality of LED chips mounted on the metal wiring portion, a power receiving terminal connected to the metal wiring portion and used for connection with another LED sheet, and a power feeding terminal connected to the metal wiring portion and used for connection with another LED sheet.

According to a second aspect of the present disclosure, in the LED sheet according to the above-described first aspect, the LED sheet may further include a power supply wiring that connects the metal wiring portion to the power receiving terminal and the power feeding terminal.

According to a third aspect of the present disclosure, in the LED sheet according to the above-described second aspect, a thickness of the power supply wiring may be greater than or equal to 18 $\mu$m and less than or equal to 100 $\mu$m, and a line width of the power supply wiring may be greater than or equal to 5 mm and less than or equal to 30 mm.

According to a fourth aspect of the present disclosure, in the LED sheet according to any one of the above-described first aspect to the above-described third aspect, the power receiving terminal and the power feeding terminal may be electric conductors, and at least part of the power receiving terminal and at least part of the power feeding terminal may be exposed.

According to a fifth aspect of the present disclosure, in the LED sheet according to any one of the above-described first aspect to the above-described fourth aspect, the power receiving terminal and the power feeding terminal may be allowed to be folded back to the first surface side or the second surface side.

According to a sixth aspect of the present disclosure, in the LED sheet according to any one of the above-described first aspect to the above-described fifth aspect, the substrate may include a protruding portion that protrudes outward, and at least one of the power receiving terminal and the power feeding terminal may be formed on the protruding portion.

According to a seventh aspect of the present disclosure, in the LED sheet according to any one of the above-described first aspect to the above-described sixth aspect, the LED sheet may include an indicating part that displays an ON state of the LED chips and an OFF state of the LED chips.

According to an eighth aspect of the present disclosure, in the LED sheet according to any one of the above-described first aspect to the above-described seventh aspect, the substrate may have flexibility.

According to a ninth aspect of the present disclosure, in the LED sheet according to any one of the above-described first aspect to the above-described eighth aspect, a planar shape of the substrate may be a k-sided polygon (where k is a natural number greater than or equal to three), a plurality of the power feeding terminals may be provided, and the power feeding terminals may be provided at positions respectively corresponding to at least two sides among k sides of the k-sided polygon.

According to a tenth aspect of the present disclosure, in the LED sheet according to the above-described ninth aspect, k-1 or less number of the power feeding terminals may be provided, and the power feeding terminals may be provided at position respectively corresponding to sides different from each other among the k sides of the k-sided polygon.

An eleventh aspect of the present disclosure is an LED sheet assembly. The LED sheet assembly includes a first LED sheet and a second LED sheet connected to the first LED sheet. Each of the first LED sheet and the second LED sheet is the LED sheet according to any of the above-described first aspect to the above-described tenth aspect, and the power feeding terminal of the first LED sheet may be connected to the power receiving terminal of the second LED sheet.

According to a twelfth aspect of the present disclosure, in the LED sheet assembly according to the above-described eleventh aspect, the power feeding terminal of the first LED sheet and the power receiving terminal of the second LED sheet may be connected in parallel with each other.

According to a thirteenth aspect of the present disclosure, the LED sheet assembly according to the above-described eleventh aspect or the above-described twelfth aspect may further include a connecting member attached to the first LED sheet and the second LED sheet, and the power feeding terminal of the first LED sheet and the power receiving terminal of the second LED sheet may be connected to each other via the connecting member.

According to a fourteenth aspect of the present disclosure, in the LED sheet assembly according to any one of the above-described eleventh aspect to the above-described thirteenth aspect, the LED sheet assembly may further include a connecting member attached to the first LED sheet and the second LED sheet, and the second LED sheet may be connected to the first LED sheet via the connecting member.

According to a fifteenth aspect of the present disclosure, in the LED sheet assembly according to any one of the above-described eleventh aspect to the above-described fourteenth aspect, the LED sheet assembly may be foldable.

According to a sixteenth aspect of the present disclosure, in the LED sheet assembly according to any one of the above-described eleventh aspect to the above-described fifteenth aspect, the power receiving terminal and the power feeding terminal may be covered with an insulator.

A seventeenth aspect of the present disclosure is a culture system. The culture system includes a culture tube that cultures algae, and the LED sheet assembly according to any one of the above-described eleventh aspect to the above-described sixteenth aspect, covering an outer surface of the culture tube.

According to the present embodiment, the LED sheets can be easily connected to each other.

Brief Description of Drawings

[Fig. 1] Fig. 1 is a perspective view showing a culture system according to an embodiment.

[Fig. 2] Fig. 2 is a vertical sectional view showing the culture system according to the embodiment.

[Fig. 3] Fig. 3 is a schematic diagram showing an LED sheet assembly of the culture system according to the embodiment.

[Fig. 4] Fig. 4 is a plan view showing an LED sheet of the culture system according to the embodiment.

[Fig. 5A] Fig. 5A is a plan view showing a modification of the LED sheet of the culture system.

[Fig. 5B] Fig. 5B is a plan view showing a modification of the LED sheet of the culture system.

[Fig. 6A] Fig. 6A is a graph showing the relationship between time and voltage in the case where a direct-current constant voltage is applied from a controller to the LED sheet.

[Fig. 6B] Fig. 6B is a graph showing the relationship between time and voltage in the case where a pulse is applied to the LED sheet as a comparative example.

[Fig. 7] Fig. 7 is a sectional view (sectional view taken along the line VII-VII in Fig. 4) showing the LED sheet of the culture system according to the embodiment.

[Fig. 8] Fig. 8 is a sectional view (sectional view taken along the line VIII-VIII in Fig. 3) showing the LED sheet of the culture system according to the embodiment.

[Fig. 9] Fig. 9 is an expansion plan showing the LED sheet assembly according to an embodiment.

[Fig. 10] Fig. 10 is a sectional view (sectional view taken along the line X-X in Fig. 9) showing the LED sheet assembly according to the embodiment.

[Fig. 11] Fig. 11 is a sectional view (sectional view corresponding to Fig. 10) showing an LED sheet assembly according to an embodiment.

[Fig. 12] Fig. 12 is a plan view showing an adhesive member of the culture system according to the embodiment.

[Fig. 13A] Fig. 13A is a sectional view showing a manufacturing method for the LED sheet of the culture system according to the embodiment.

[Fig. 13B] Fig. 13B is a sectional view showing the manufacturing method for the LED sheet of the culture system according to the embodiment.

[Fig. 13C] Fig. 13C is a sectional view showing the manufacturing method for the LED sheet of the culture system according to the embodiment.

[Fig. 13D] Fig. 13D is a sectional view showing the manufacturing method for the LED sheet of the culture system according to the embodiment.

[Fig. 13E] Fig. 13E is a sectional view showing the manufacturing method for the LED sheet of the culture system according to the embodiment.

[Fig. 13F] Fig. 13F is a sectional view showing the manufacturing method for the LED sheet of the culture system according to the embodiment.

[Fig. 13G] Fig. 13G is a sectional view showing the manufacturing method for the LED sheet of the culture system according to the embodiment.

[Fig. 13H] Fig. 13H is a sectional view showing the manufacturing method for the LED sheet of the culture system according to the embodiment.

[Fig. 14] Fig. 14 is a schematic perspective view showing an example of use of the culture system according to the embodiment.

[Fig. 15] Fig. 15 is a schematic perspective view showing an example of use of the culture system according to the embodiment.

[Fig. 16] Fig. 16 is a schematic diagram showing a first modification of the LED sheet assembly according to the embodiment.

[Fig. 17] Fig. 17 is a schematic diagram showing a second modification of the LED sheet assembly according to the embodiment.

[Fig. 18] Fig. 18 is a sectional view (sectional view taken along the line XVIII-XVIII in Fig. 17) showing the second modification of the LED sheet assembly according to the embodiment.

[Fig. 19] Fig. 19 is a schematic expansion plan showing a third modification of the LED sheet assembly according to the embodiment.

[Fig. 20A] Fig. 20A is a schematic perspective view showing another example of the third modification of the LED sheet assembly according to the embodiment.

[Fig. 20B] Fig. 20B is a schematic perspective view showing another example of the third modification of the LED sheet assembly according to the embodiment.

[Fig. 20C] Fig. 20C is a schematic perspective view showing another example of the third modification of the LED sheet assembly according to the embodiment.

[Fig. 20D] Fig. 20D is a schematic perspective view showing another example of the third modification of the LED sheet assembly according to the embodiment.

[Fig. 20E] Fig. 20E is a schematic perspective view showing another example of the third modification of the LED sheet assembly according to the embodiment.

[Fig. 20F] Fig. 20F is a schematic perspective view showing another example of the third modification of the LED sheet assembly according to the embodiment.

[Fig. 21] Fig. 21 is a schematic diagram showing another example of the third modification of the LED sheet assembly according to the embodiment.

[Fig. 22] Fig. 22 is a schematic diagram showing another example of the third modification of the LED sheet assembly according to the embodiment.

[Fig. 23] Fig. 23 is a sectional view (sectional view corresponding to Fig. 18) showing a fourth modification of the LED sheet assembly according to the embodiment.

[Fig. 24] Fig. 24 is a perspective view showing a culture system including a fifth modification of the LED sheet assembly according to the embodiment.

[Fig. 25A] Fig. 25A is a schematic perspective view showing a folding method for the LED sheet assemblies according to the embodiment and the modifications.

[Fig. 25B] Fig. 25B is a schematic perspective view showing another example of the folding method for the LED sheet assemblies according to the embodiment and the modifications.

[Fig. 25C] Fig. 25C is a schematic perspective view showing another example of the folding method for the LED sheet assemblies according to the embodiment and the modifications.

[Fig. 25D] Fig. 25D is a schematic perspective view showing another example of the folding method for the LED sheet assemblies according to the embodiment and the modifications.

[Fig. 25E] Fig. 25E is a schematic perspective view showing another example of the folding method for the LED sheet assemblies according to the embodiment and the modifications.

[Fig. 25F] Fig. 25F is a schematic perspective view showing another example of the folding method for the LED sheet assemblies according to the embodiment and the modifications.

Description of Embodiments

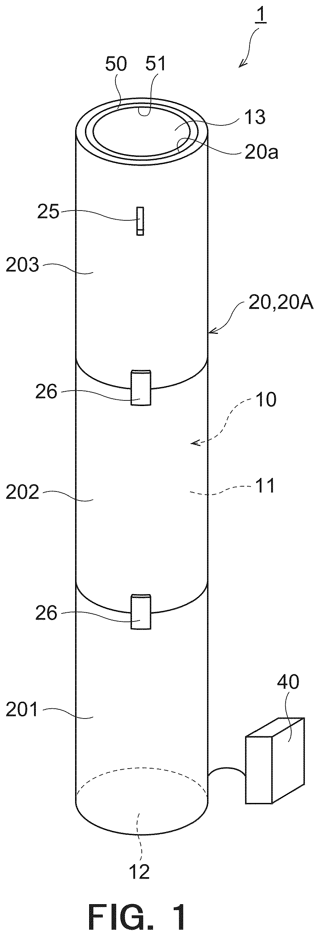

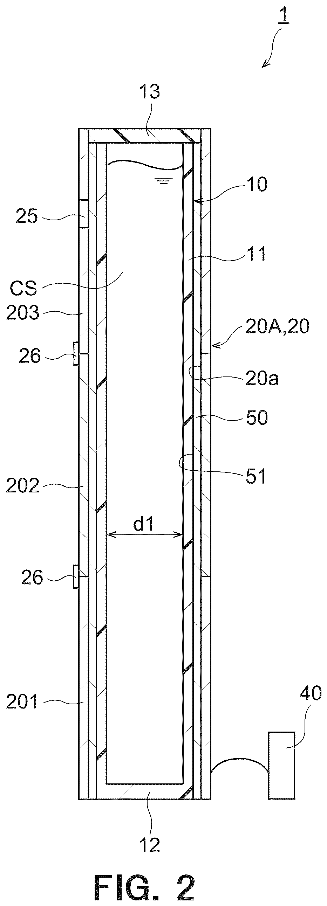

Hereinafter, an embodiment will be specifically described with reference to the drawings. The drawings described below are schematically shown drawings. Therefore, the size and shape of each portion are exaggerated as needed for the purpose of easy understanding. The embodiment may be modified as needed without departing from the technical idea. In the drawings described below, like reference signs denote the same portions, and the detailed description may be partially omitted. The numeric values, such as dimensions, and material names of members described in the specification are illustrative as the embodiment and are not limited thereto. The numeric values and material names may be selected and used as needed. In the specification, terms that specify shapes and geometrical conditions, for example, terms, such as parallel, orthogonal, and perpendicular, are interpreted as not only the strict senses of the terms but also substantially the same states. In the specification, "upper" and "lower" respectively refer to the upper side and lower side in the state where a culture tube is in an upright position (Figs. 1 and 2).

(Culture System)

As shown in Figs. 1 and 2, a culture system 1 includes a culture tube 10 that cultures algae and an LED sheet assembly 20A that covers the outer surface of the culture tube 10. The LED sheet assembly 20A includes a plurality of LED sheets 20. A controller 40 is electrically connected to one of the plurality of LED sheets 20.

The culture tube 10 includes a hollow trunk portion 11, a bottom portion 12 provided at the lower side of the trunk portion 11, and a cap portion 13 provided at the upper side of the trunk portion 11. The trunk portion 11 has a cylindrical shape. The trunk portion 11 may have a polygonal tubular shape, such as a quadrilateral tubular shape or an octagonal tubular shape.

The bottom portion 12 has a circular shape in plan view and is connected to the lower end of the trunk portion 11. The bottom portion 12 may be molded integrally with the trunk portion 11 or may be detachably attached to the trunk portion 11. The planar shape of the bottom portion 12 may be an elliptical shape or a polygonal shape, such as a quadrilateral shape.

The cap portion 13 has a circular shape in plan view and is connected to the upper end of the trunk portion 11. The cap portion 13 may be detachably attached to the trunk portion 11. The planar shape of the cap portion 13 may be an elliptical shape or a polygonal shape, such as a quadrilateral shape.

The above culture tube 10 is filled with a culture solution CS. Examples of the material of the culture tube 10 include glass (refractive index: approximately 1.55), polycarbonate (refractive index: approximately 1.58), and resin materials such as acrylic.

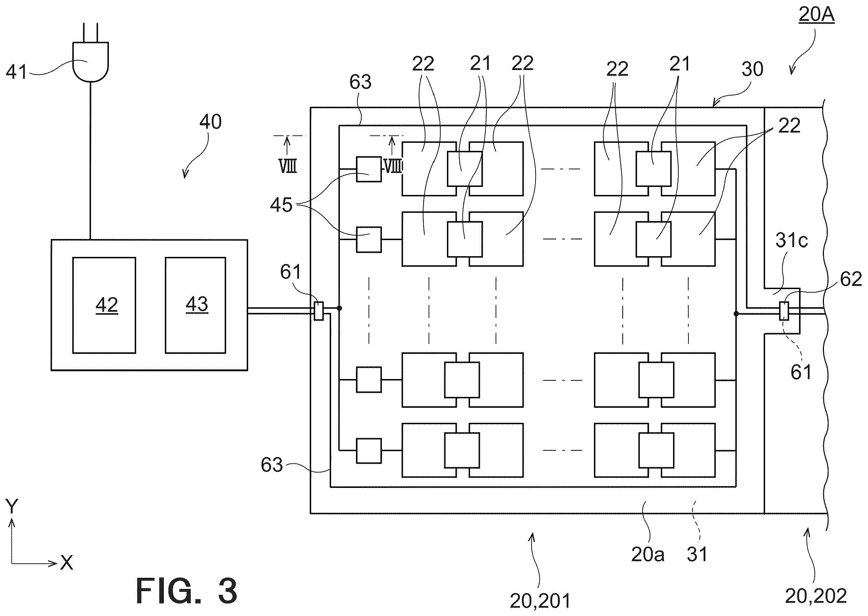

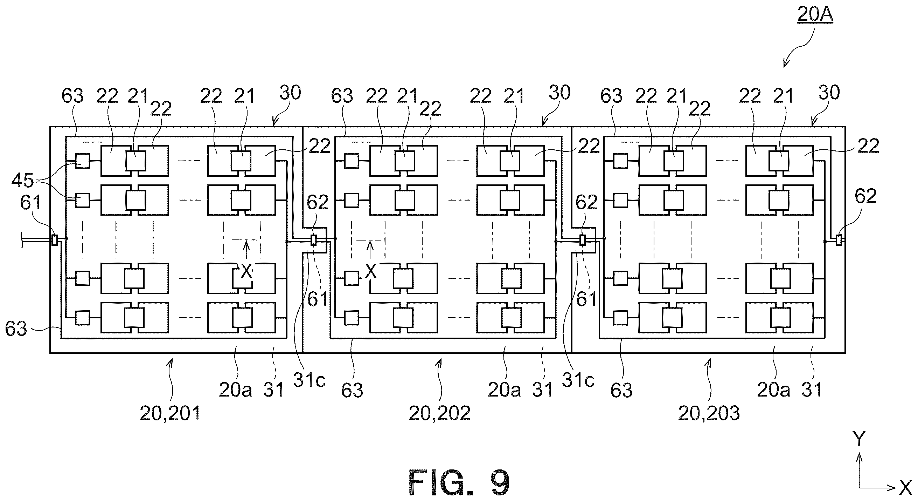

Next, the LED sheet assembly 20A will be described in detail. As described above, the LED sheet assembly 20A includes the plurality of LED sheets 20. In the illustrated example, the LED sheet assembly 20A includes a first LED sheet 201, a second LED sheet 202, and a third LED sheet 203 (see Figs. 1 and 2). Of these, the second LED sheet 202 is connected to the first LED sheet 201. The third LED sheet 203 is connected to the second LED sheet 202. In the present embodiment, the plurality of LED sheets 20 (the first LED sheet 201, the second LED sheet 202, and the third LED sheet 203) is arranged in a first array direction (X direction, see Figs. 3 and 9) of LED chips 21. The LED sheet assembly 20A may be foldable as will be described later. In the specification, the first LED sheet 201, the second LED sheet 202, or the third LED sheet 203 is also simply referred to as LED sheet 20. The LED sheet assembly 20A may include four or more LED sheets 20.

The LED sheet assembly 20A further includes connecting members 26. In the illustrated example, the LED sheet assembly 20A includes a plurality of (two) connecting members 26. The number of the connecting members 26 is not limited thereto. For example, although not illustrated, the LED sheet assembly 20A may include only one connecting member 26, or the LED sheet assembly 20A may include three or more connecting members 26. In the present embodiment, one of the two connecting members 26 is detachably attached to the first LED sheet 201 and the second LED sheet 202. The second LED sheet 202 is connected to the first LED sheet 201 via the connecting member 26. The other one of the connecting members 26 is detachably attached to the second LED sheet 202 and the third LED sheet 203. The third LED sheet 203 is connected to the second LED sheet 202 via the connecting member 26. The connecting member 26 may be attached to the first LED sheet 201 or the like in a manner such that the connecting member 26 cannot be detached from the first LED sheet 201 or the like.

The connecting member 26 may be, for example, tape such as double-sided tape and hook and loop fasteners, adhesive, or rivets. The connecting member 26 may be a cord-like member such as cable tie. When the connecting member 26 is a cable-like member, for example, through holes (not shown) that respectively penetrate the LED sheets 20 may be formed, and the LED sheets 20 may be connected to each other by passing the connecting member 26 through the through holes. In this case, the area around the through hole may be protected with a grommet (not shown). In the illustrated example, the connecting members 26 connect the LED sheets 20 on the side opposite to light emitting surfaces 20a of the LED sheets 20; however, the configuration is not limited thereto. The connecting members 26 may connect the LED sheets 20 on the side of the light emitting surfaces 20a, or the connecting members 26 may also connect the LED sheets 20 on the side of the light emitting surfaces 20a and on the side opposite to the light emitting surfaces 20a.

The LED sheet assembly 20A is wrapped around the culture tube 10 such that the light emitting surface 20a of each LED sheet 20 faces the culture tube 10. The LED sheets 20 cover the trunk portion 11 of the culture tube 10. The LED sheets 20 preferably cover the entire area of the trunk portion 11 of the culture tube 10 in the circumferential direction of the trunk portion 11. Thus, light can be applied from all around the circumference of the trunk portion 11 into the culture tube 10. Therefore, it is possible to enhance the growth efficiency of algae. The LED sheets 20 preferably cover the entire area of the trunk portion 11 of the culture tube 10 in the longitudinal direction of the trunk portion 11. Thus, it is possible to further enhance the utilization efficiency of light.

Here, the LED sheets 20 include an indicating part 25 that indicates the ON state of the LED chips 21 (described later) or the OFF state of the LED chips 21. Thus, even when the LED sheets 20 are wrapped around the culture tube 10, it is possible to easily check whether the LED chips 21 are in the ON state or in the OFF state. In the present embodiment, the indicating part 25 is a through hole that penetrates the LED sheet 20. In this case, when the LED chips 21 are lit, light slightly leaks from the indicating part 25. On the other hand, when the LED chips 21 are not lit, no light leaks from the indicating part 25. In this way, the LED sheets 20 are configured so that the ON state or the OFF state of the LED chips 21 can be easily checked.

When the indicating part 25 is formed, only the substrate 31 (described later) may be left without forming a through hole in the LED sheet 20. In this case as well, since the substrate 31 is transparent, it is possible to easily check whether the LED chips 21 are in the ON state or in the OFF state. In the illustrated example, the shape of the through hole is a rectangular shape in front view; however, the size and shape of the through hole are not limited. A plurality of through holes may be formed. For example, a through hole may be formed one by one near each LED chip 21. Thus, it is possible to easily identify a broken LED chip 21.

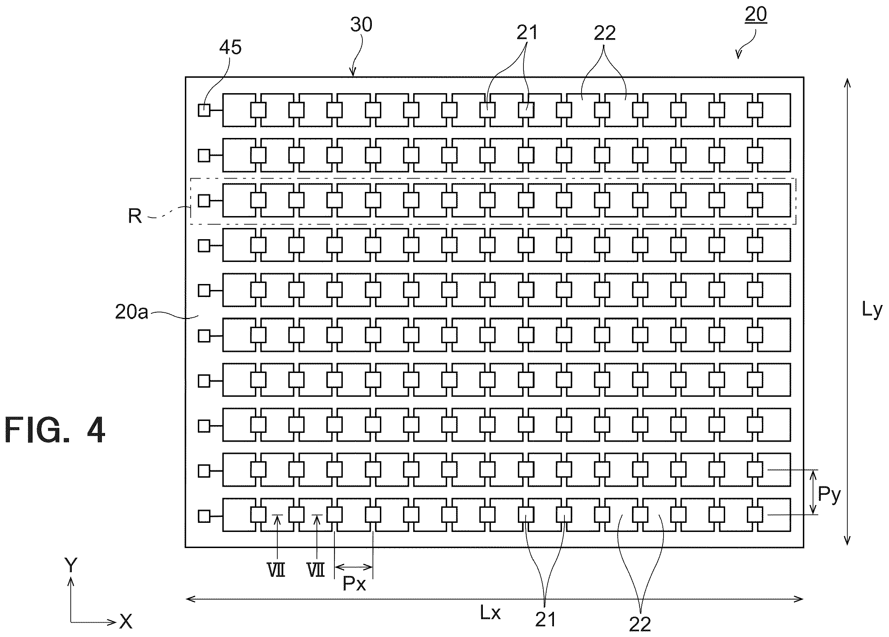

As shown in Figs. 3 and 4, the LED sheet 20 is a so-called single-sided light emission-type surface light source sheet. A plurality of LED chips 21 is arranged on the side of the light emitting surface 20a of this LED sheet 20. With the use of such a direct-lit LED sheet 20, irradiation light from the LED chips 21 directly passes through the light emitting surface 20a. For this reason, the intensity of irradiation light can be increased, so the culture of algae can be accelerated. The LED sheet 20 can have a thinner overall thickness compared to an LED bar light, so it is possible to suppress the occurrence of shadows on the side part of the LED chips 21.

The LED sheet 20 of Fig. 4 includes a flexible wiring substrate 30, a plurality of LED chips 21 mounted on a metal wiring portion 32 (described later) of the flexible wiring substrate 30, a power receiving terminal 61, and a power feeding terminal 62. By using the flexible wiring substrate 30, the LED sheet 20 with a sheet surface having a relatively large area can be obtained. The LED sheet 20 with a sheet surface having a relatively large area can reduce the number of the LED sheets 20 to be used, so variation in the amount of light that can occur due to the placement of the plurality of LED sheets 20 can be suppressed. The LED sheet 20 with a sheet surface having a relatively large area may have, for example, a size of about 600 mm x 500 mm. In Fig. 4, a light reflection insulating protective film 34 and a transparent protective film 35 (described later) are not shown.

In this case, the LED chips 21 are regularly arranged on the flexible wiring substrate 30. Specifically, the LED chips 21 are arranged in a grid point manner in plan view in the flexible wiring substrate 30. In other words, the LED chips 21 are arranged in a matrix of multiple rows and multiple columns, and N rows R of M LED chips 21 connected in series are arranged.

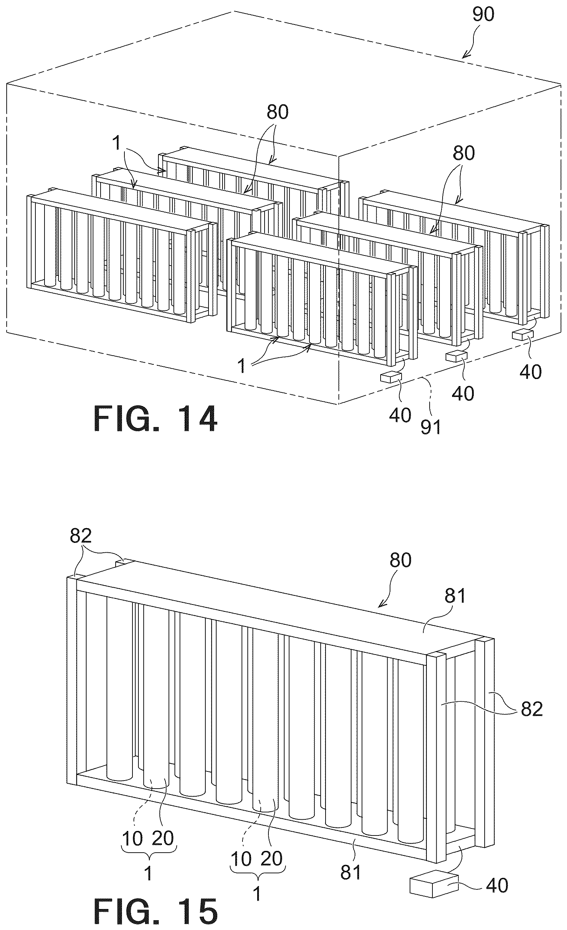

For example, in Fig. 4, 14 (M = 14) LED chips 21 are connected in series in the first array direction (X direction) of the LED chips 21. Furthermore, 10 rows R (N = 10) of the 14 LED chips 21 are arranged in parallel in a second array direction (Y direction) of the LED chips 21. The number of LED chips 21 arranged is not limited thereto. Specifically, six or more (M ≥ 6) LED chips 21 may be arranged in series in the first array direction (X direction) or ten or more (M ≥ 10) LED chips 21 may be arranged in series in the first array direction (X direction). Fourteen or less (14 ≥ M) LED chips 21 may be arranged in series in the first array direction (X direction), or 12 or less (12 ≥ M) LED chips 21 may be arranged in series in the first array direction (X direction). Four or more rows R (N ≥ 4) may be arranged in parallel in the second array direction (Y direction) of the LED chips 21, and six or more rows R (N ≥ 6) are preferably arranged in parallel in the second array direction (Y direction) of the LED chips 21. Furthermore, 10 or less rows R (10 ≥ N) may be arranged in parallel in the second array direction (Y direction) of the LED chips 21, and eight or less rows R (8 ≥ N) may be arranged in parallel in the second array direction (Y direction) of the LED chips 21. When 10 or more LED chips 21 are arranged in series, the pitch of the LED chips 21 in the first array direction (X direction) can be shortened. Thus, in-plane variation in illuminance of the LED sheet 20 is suppressed. Therefore, it is possible to suppress variation in light to be applied to the culture tube 10. When 14 or less LED chips 21 are arranged in series, power consumption can be reduced. When four or more rows of LED chips 21 are arranged in parallel in the second array direction (Y direction) of the LED chips 21, it is possible to suppress influences on the LED chips 21 in other rows even in the case of a break of a specific one or some of the LED chips 21. Thus, it is possible to suppress an extreme decrease in the illuminance of the overall LED sheet 20. By the way, when the LED sheet 20 is a direct-lit type, there is a higher possibility that a worker comes into contact with LED chips 21 during the installation or removal of the LED sheet 20. When a worker accidentally makes strong contact with LED chips 21, there is a high risk that the LED chips 21 break. For this reason, taking measures in case LED chips 21 break is important from the viewpoint of risk management. Furthermore, when 10 or less rows of LED chips 21 are arranged in parallel, power consumption can be reduced.

The LED sheet 20 includes a plurality of metal wiring portions 22. The plurality of metal wiring portions 22 is arranged in the first array direction (X direction). The plurality of metal wiring portions 22 arranged in the first array direction (X direction) is in correspondence with each row R of the LED chips 21. Each of the LED chips 21 is placed so as to bridge a pair of the metal wiring portions 22 adjacent to each other in the X direction. The terminals (not shown) of each LED chip 21 are respectively electrically connected to the pair of metal wiring portions 22. The plurality of metal wiring portions 22 makes up a power feeding portion to the LED chips 21. When electric power is supplied to the plurality of metal wiring portions 22, all the LED chips 21 arranged in the row R light up. The plurality of metal wiring portions 22 partially makes up the metal wiring portion 32 (described later).

The pitch Px of the LED chips 21 in the first array direction (X direction) may be greater than or equal to 15 mm and is preferably greater than or equal to 25 mm. The pitch Px of the LED chips 21 may be less than or equal to 100 mm and is preferably less than or equal to 60 mm. The pitch Py of the LED chips 21 in the second array direction (Y direction) may be greater than or equal to 15 mm and is preferably greater than or equal to 25 mm. The pitch Py of the LED chips 21 may be less than or equal to 100 mm and is preferably less than or equal to 75 mm. By setting the pitches of the LED chips 21 to the above ranges, the brightness of the LED sheet 20 can be made uniform in the plane. Therefore, variation in light to be applied to a space is reduced, and the power consumption of the LED sheet 20 can be suppressed.

The thickness of the thickest part within the LED sheet 20 is preferably less than or equal to 5 mm. By reducing the thickness of the LED sheet 20 in this way, it is possible to suppress the bulk of the LED sheet 20 when the LED sheet 20 is attached to the culture tube 10. Thus, even with a limited space, the LED sheet 20 can be easily attached to the culture tube 10.

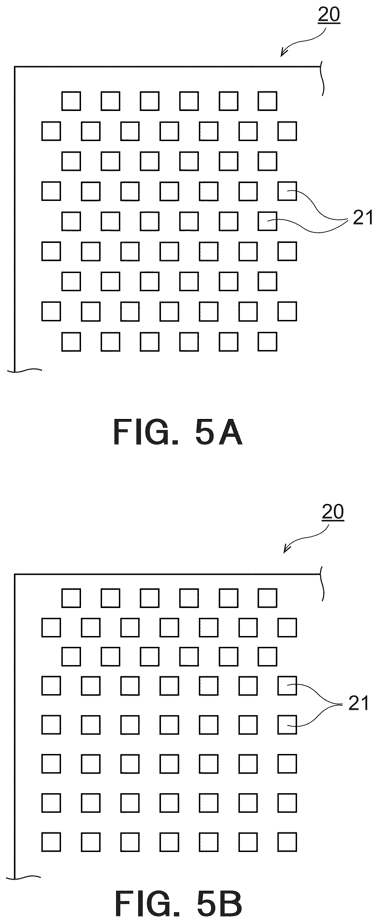

The arrangement of the LED chips 21 is not limited to a grid point manner in plan view. Alternatively, as shown in Fig. 5A, the LED chips 21 may be arranged in a staggered manner in plan view. Alternatively, the LED chips 21 do not need to be arranged uniformly in the plane of the LED sheet 20. For example, the density of the LED chips 21 may be increased at the peripheral part of the LED sheet 20. Specifically, as shown in Fig. 5B, the LED chips 21 may be arranged in a grid point manner at the center part (the lower part in Fig. 5B) of the LED sheet 20, and the LED chips 21 may be arranged in a staggered manner at the peripheral part (the upper part in Fig. 5B) of the LED sheet 20. Thus, a decrease in the brightness of the LED sheet 20 at the peripheral part of the LED sheet 20 can be suppressed, and variation in light to be applied to a space is reduced by making the brightness of the LED sheet 20 uniform in the plane.

The overall shape of the LED sheet 20 is a rectangular shape in plan view; however, the size and planar shape of the LED sheet 20 are not limited. The LED sheet 20 has a high degree of flexibility in size and shape processing, the LED sheet 20 is able to flexibly meet various demands in terms of these points. Since the LED sheet 20 has flexibility, the LED sheet 20 can be attached to not only installation surfaces having flat shapes but also installation surfaces having various shapes.

In Fig. 4, the length Lx of the LED sheet 20 in the first array direction (X direction) is preferably greater than or equal to 500 mm and more preferably greater than or equal to 550 mm. The length Lx of the LED sheet 20 in the first array direction (X direction) is preferably less than or equal to 750 mm and more preferably less than or equal to 650 mm. The length Ly of the LED sheet 20 in the second array direction (Y direction) is preferably greater than or equal to 300 mm and more preferably greater than or equal to 350 mm. The length Ly of the LED sheet 20 in the second array direction (Y direction) is preferably less than or equal to 500 mm and more preferably less than or equal to 450 mm. Since the size of each individual LED sheet 20 is not excessively small, the amount of light from the LED sheet 20 can be increased. Since the size of each individual LED sheet 20 is not excessively large, influences on the other LED chips 21 can be minimized when a specific one or some of the LED chips 21 break. For this reason, an extreme decrease in the illuminance of the overall LED sheet 20 can be suppressed, and the range in which the illuminance decreases can be limited.

Next, the controller 40 will be described. As shown in Fig. 3, the controller 40 supplies electric power to the LED sheet 20 and controls light emission and the like of the LED sheet 20. The controller 40 is detachably connected to the LED sheet 20 via a connector (not shown) provided on the LED sheet 20. In other words, the controller 40 is provided separately from the LED sheet 20 and is externally connected to the LED sheet 20. In other words, the controller 40 is not integrated with the LED sheet 20. With this configuration, the controller 40 can be separated from the LED sheet 20, and the controller 40 can be installed in any location. Therefore, even with a limited space, the LED sheet 20 can be easily attached to the culture tube 10.

The controller 40 includes a power input section 41, an AC/DC converter (driver) 42, and a PWM control section 43. Of these, for example, alternating-current voltage having a selected voltage of 100 V to 240 V is supplied to the power input section 41. The AC/DC converter 42 converts the alternating-current voltage of 100 V to 240 V to a constant direct-current voltage (for example, 44 V). The PWM control section 43 controls the lighting of the LED chips 21 of the LED sheet 20 by changing the pulse width of a constant voltage waveform from the AC/DC converter 42 to a selected width. In other words, the PWM control section 43 also plays a role as a light control section that controls the lighting of the LED sheet 20. The constant voltage output from the PWM control section 43 is applied to the LED sheet 20.

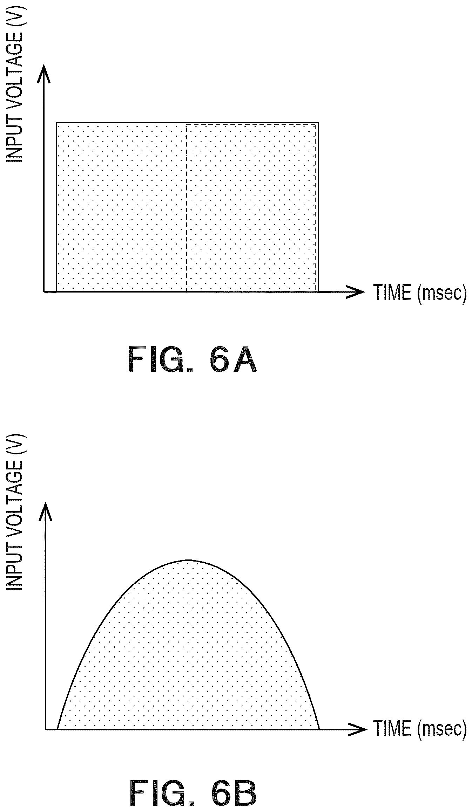

In the present embodiment, a direct-current constant voltage is applied to the LED sheet 20 from the PWM control section 43 of the controller 40. Thus, it is possible to control the lighting of the LED chips 21, unlike the case where a rectified pulse voltage is directly applied to the LED sheet 20. In other words, the PWM control section 43 is capable of controlling the illuminance of the LED chips 21 to a selected illuminance by changing the duty ratio of the direct-current voltage from the AC/DC converter 42 as needed. For example, as shown in Fig. 6A, the PWM control section 43 is capable of reducing the illuminance of the LED chips 21 by reducing the duty ratio of the constant voltage from the AC/DC converter 42 from 100% (continuous line) to 50% (dashed line).

In this way, when the illuminance of the LED chips 21 is adjusted, the illuminance of the LED sheet 20 may be adjusted according to, for example, the degree of growth of algae. Thus, it is possible to adjust the degree of growth of algae. For example, the illuminance of the LED sheet 20 may be reduced in an early growth stage in which the size of the algae is small, and the illuminance of the LED sheet 20 may be increased in a late growth stage where the size of the algae has increased and the algae have grown thick in the culture tube 10. As another example of adjusting the illuminance of the LED sheet 20, the illuminance may be increased in the case of types of algae that need a high illuminance, and the illuminance may be reduced in the case of types of algae that can be grown even at a low illuminance. The illuminance may be increased when shipping timing needs to be advanced, and the illuminance may be reduced when shipping timing needs to be delayed.

When a direct-current constant voltage is applied from the PWM control section 43 to the LED sheet 20, the accumulated amount of light per unit time from the LED sheet 20 can be increased. In other words, for example, the accumulated amount of light (the area of a shaded part in Fig. 6A) in the case where a direct-current constant voltage is applied to the LED sheet 20 can be made greater than the accumulated amount of light (the area of a shaded part in Fig. 6A) in the case where a voltage is applied in pulse form as a comparative example. With this configuration, the luminous efficiency of light from the LED sheet 20 is enhanced, and the growth efficiency of algae can be improved.

Referring again to Fig. 3, the LED sheet 20 includes regulators 45. In this case, the regulators 45 are respectively provided in correspondence with the rows of the LED chips 21, and specifically 10 regulators 45 are provided in correspondence with 10 rows of the LED chips 21. Each of the regulators 45 plays a role in maintaining a constant current flowing through the plurality of LED chips 21 of the associated row. With this configuration, even when one of the LED chips 21 breaks, flow of an excessive current through the LED chips 21 in the other rows is suppressed, so a break of the LED chips 21 in the other rows can be suppressed. As a result, an extreme decrease in the illuminance of the overall LED sheet 20 can be suppressed, so variation in light to be applied to a space can be reduced.

As described above, the LED sheet 20 includes the power receiving terminal 61 and the power feeding terminal 62. Each of the power receiving terminal 61 and the power feeding terminal 62 is placed on the surface 31a of the substrate 31 and connected to the metal wiring portion 32 (described later). The power receiving terminal 61 and the power feeding terminal 62 are terminals used when the LED sheet 20 is connected to another LED sheet 20. Specifically, the power receiving terminal 61 is a terminal for receiving electric power supplied from the controller 40 or another LED sheet 20. The power feeding terminal 62 is a terminal for supplying electric power to another LED sheet 20. The details of the power receiving terminal 61 and the power feeding terminal 62 will be described later.

(Members of LED Sheet)

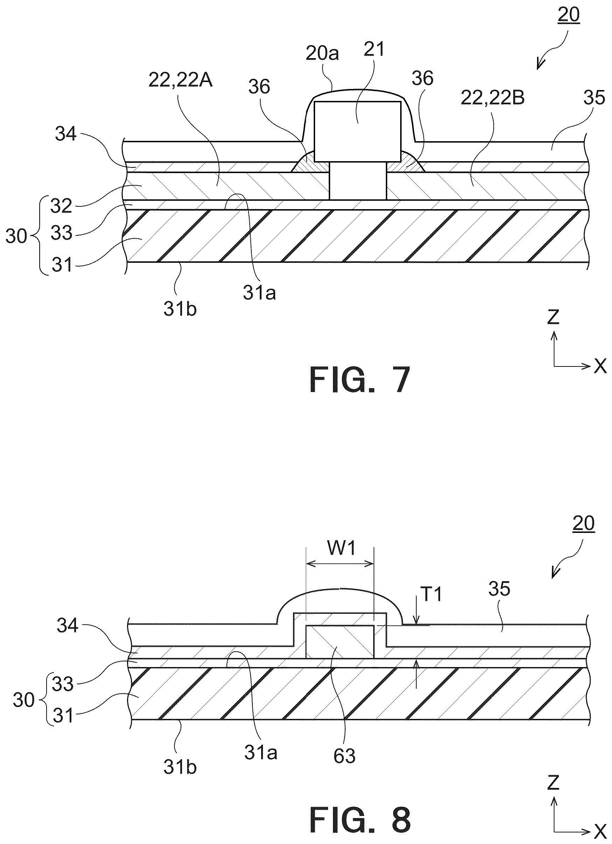

Next, members that make up the LED sheet 20 will be described. As shown in Fig. 7, the LED sheet 20 includes the flexible wiring substrate 30, and the plurality of LED chips 21 placed on the flexible wiring substrate 30. Of these, the flexible wiring substrate 30 includes the substrate 31 and the metal wiring portion 32 on the substrate 31. The metal wiring portion 32 is laminated on the substrate 31 via an adhesive layer 33. The substrate 31 has the surface (first surface) 31a on the side of the light emitting surface 20a and a back surface (second surface) 31b positioned on the side opposite to the surface 31a.

Each of the LED chips 21 is mounted so as to be able to establish electrical continuity with the metal wiring portion 32. In the LED sheet 20, since the LED chips 21 are mounted on the flexible wiring substrate 30, the plurality of LED chips 21 can be placed with a desired high density.

The metal wiring portion 32 is positioned on the surface 31a of the substrate 31. The light reflection insulating protective film 34 is formed on the metal wiring portion 32. The light reflection insulating protective film 34 is not formed in the region within the LED sheet 20 where the LED chips 21, the regulators 45, the power receiving terminal 61, or the power feeding terminal 62 is provided. The light reflection insulating protective film 34 is not formed in the peripheral region of the region where the LED chips 21, the regulators 45, the power receiving terminal 61, or the power feeding terminal 62 is provided. The light reflection insulating protective film 34 is a layer having an electrically insulating function that contributes to improvement in the migration resistance properties of the LED sheet 20 and a light reflection function that contributes to improvement in light environment created by the LED sheet 20. This layer is made of an electrically insulating resin composition containing a white pigment. When the migration resistance properties and the light reflection function are obtained by the above-described metal wiring portion 32 and the transparent protective film 35 (described later), the light reflection insulating protective film 34 does not need to be formed on the metal wiring portion 32.

The transparent protective film 35 is formed so as to cover the light reflection insulating protective film 34 and the LED chips 21. The transparent protective film 35 is a resin film formed at the outermost surface (the surface closest to the light emitting surface 20a side) of the LED sheet 20 to mainly ensure the waterproofness of the LED sheet 20.

Solder portions 36 are provided on the metal wiring portion 32. Each of the LED chips 21 is electrically connected to the metal wiring portion 32 via the solder portions 36.

Referring again to Fig. 3, the LED sheet 20 further includes a power supply wiring 63 that connects the metal wiring portion 32 to the power receiving terminal 61 and the power feeding terminal 62. The power supply wiring 63 is branched off from the power receiving terminal 61. The power supply wiring 63 is placed on the surface 31a of the substrate 31 and is placed outside the LED chips 21 so as to surround the 10 rows R of LED chips. The power supply wiring 63 is electrically connected to the power supply wiring 63 of another LED sheet 20 having the same configuration as the LED sheet 20. In other words, the power supply wiring 63 is detachably connected to the power supply wiring 63 of another LED sheet 20 via the power feeding terminal 62 and another power receiving terminal 61 of that LED sheet 20. A current from the power supply wiring 63 is supplied to another LED sheet 20 via the power feeding terminal 62 and the power receiving terminal 61 of that LED sheet 20. With this configuration, two LED sheets 20 can be connected, and these two LED sheets 20 can be controlled at the same time by the single controller 40. Since the single controller 40 is able to control the plurality of LED sheets 20 at the same time, the number of controllers 40 can be reduced, so it is possible to suppress the bulk of the culture system 1. Therefore, even with a limited space, the culture system 1 can be easily installed.

(Substrate)

In the present embodiment, the substrate 31 has flexibility. A resin film having flexibility may be used as the substrate 31. In the specification, the phrase "having flexibility" means that "the radius of curvature when bent is at least less than or equal to 1 m, preferably 50 cm, more preferably 30 cm, further preferably 10 cm, and particularly preferably 5 cm."

In the present embodiment, the substrate 31 includes a protruding portion 31c that protrudes outward. The power feeding terminal 62 is formed on the protruding portion 31c.

The planar shape of the substrate 31 may be substantially the same as the overall planar shape of the LED sheet 20 or may be quadrilateral (rectangular) (see Fig. 3). However, the planar shape of the substrate 31 is not limited. The planar shape of the substrate 31 may be, for example, a k-sided polygon (where k is a natural number greater than or equal to three). There is no upper limit of k; however, for example, the upper limit of k may be less than or equal to eight or may be less than or equal to six. In the specification, the planar shape of the substrate 31 means the planar shape of the region within the substrate 31, excluding the protruding portion 31c where the power receiving terminal 61 or the power feeding terminal 62 is formed.

A high heat resistant, electrically insulating thermoplastic resin may be used as the material of the substrate 31. A polyimide resin (PI) or polyethylene naphthalate (PEN) having high heat resistance, dimensional stability during heating, mechanical strength, and durability may be used as such a resin. Of these, polyethylene naphthalate (PEN) having improved heat resistance and dimensional stability imparted through a heat resistance improving treatment, such as annealing, is preferably used. Polyethylene terephthalate (PET) having improved flame resistance imparted by adding a flame-resistant inorganic filler or the like may be used.

The surface 31a of the substrate 31 faces the culture tube 10. The surface 31a is preferably colored white. Thus, it is possible to effectively reflect light that leaks from the culture tube 10 to the outside, so the utilization efficiency of light can be further improved.

The thickness of the substrate 31 is not limited. The thickness of the substrate 31 is preferably greater than or equal to about 10 $\mu$m and more preferably greater than or equal to 50 $\mu$m, from the viewpoint that the substrate 31 does not become a bottleneck as a heat dissipation path, has heat resistance and electrical insulation properties, and balances with manufacturing costs. The thickness of the substrate 31 is preferably less than or equal to 500 $\mu$m and more preferably less than or equal to 250 $\mu$m. From the viewpoint of maintaining good productivity in the case of manufacturing by a roll-to-roll process as well, the thickness of the substrate 31 preferably falls within the above range.

(Adhesive Layer)

A known resin adhesive may be used as needed for an adhesive that forms the adhesive layer 33. Of those resin adhesives, a urethane adhesive, a polycarbonate adhesive, a silicone adhesive, an ester adhesive, an epoxy adhesive, or the like may be particularly preferably used.

(Metal Wiring Portion)

The metal wiring portion 32 is a wiring pattern formed on the surface 31a (the surface on the side of the light emitting surface 20a) of the substrate 31 from a conductive base material, such as metal foil. The metal wiring portion 32 is preferably formed on the surface 31a of the substrate 31 via the adhesive layer 33 by dry lamination. The metal wiring portion 32 includes the above-described plurality of metal wiring portions 22. The plurality of metal wiring portions 22 includes a first metal wiring portion 22A and a second metal wiring portion 22B placed apart from the first metal wiring portion 22A. The LED chip 21 is mounted on the first metal wiring portion 22A and the second metal wiring portion 22B, and the LED chip 21 is electrically connected to the first metal wiring portion 22A and the second metal wiring portion 22B. The LED chip 21 lights up by electric power supplied to the first metal wiring portion 22A and the second metal wiring portion 22B.

The metal wiring portion 32 is preferably the one that achieves both heat dissipation and electrical conductivity at a high level, and, for example, copper foil may be used. In this case, heat dissipation from the LED chips 21 is stable and an increase in electrical resistance is suppressed, so light emission variation among the LED chips 21 reduces, with the result that stable light emission is possible. The life of the LED chips 21 is also extended. Degradation of peripheral members including the substrate 31 and the like due to heat can also be suppressed, so the product life of the LED sheet 20 can also be extended. Examples of the metal that forms the metal wiring portion 32 include metals, such as aluminum, gold, and silver, in addition to the above-described copper.

The thickness of the metal wiring portion 32 may be set as needed according to, for example, the magnitude of withstand current required from the flexible wiring substrate 30. To suppress warpage caused by heat shrinkage of the substrate 31 during reflow soldering process or the like, the thickness of the metal wiring portion 32 is preferably greater than or equal to 10 $\mu$m. On the other hand, the thickness of the metal wiring portion 32 is preferably less than or equal to 50 $\mu$m. With this configuration, sufficient flexibility of the flexible wiring substrate 30 can be maintained, so a decrease in handling or the like due to an increase in weight can also be suppressed.

(Solder Portion)

The solder portions 36 join the metal wiring portion 32 with the LED chips 21. Joining with solder may be performed with any one of two methods, that is, a reflow method and a laser method.

(LED Chip)

The LED chip 21 is a light-emitting element that uses light emission at a p-n junction part at which a p-type semiconductor and an n-type semiconductor are joined together. The LED chip 21 may have a structure such that a p-type electrode and an n-type electrode are respectively provided at the upper and lower surfaces of an element or may have a structure such that both a p-type electrode and an n-type electrode are provided at one side of an element.

The one having high luminous efficiency is preferably selected as the LED chip 21. Specifically, the one having a luminous efficiency of 150 lm/W or higher is preferably used as the LED chip 21, and the one having a luminous efficiency of 180 lm/W or higher is more preferably used as the LED chip 21. When the luminous efficiency of the LED chip 21 is enhanced to 150 lm/W or higher, the number (density) of LED chips 21 mounted can be reduced, heat generation due to Joule heat from the LED chips 21 can be reduced, with the result that the degradation of peripheral members such as the substrate 31 due to heat from the LED chips 21 can be suppressed.

The LED sheet 20 is the one in which the LED chips 21 are directly mounted on the metal wiring portion 32 capable of exercising high heat dissipation as described above. With this configuration, even in the case where the LED chips 21 are placed in high density, excessive heat generated during the lighting of the LED chips 21 can be rapidly diffused through the metal wiring portion 32. Therefore, heat can be sufficiently dissipated to the outside of the LED sheet 20 through the substrate 31, so the degradation of peripheral members such as the substrate 31 due to heat from the LED chips 21 can be suppressed.

(Light Reflection Insulating Protective Film)

As shown in Fig. 7, the light reflection insulating protective film 34 is a layer formed in a region, other than the region in which the LED chips 21 are provided or their peripheral regions. The light reflection insulating protective film 34 is a so-called resist layer to improve the migration resistance properties of the flexible wiring substrate 30 because of its sufficient electrical insulation properties. The light reflection insulating protective film 34 is a light reflection layer that has light reflectivity contributing to the improvement in the luminous brightness of the light environment created by the LED sheet 20.

The light reflection insulating protective film 34 may be formed by using various resin compositions having a urethane resin or the like as a base resin and further containing a white pigment made up of an inorganic filler, such as titanium oxide. Other than a urethane resin, an acrylic polyurethane resin, a polyester resin, a phenolic resin, or the like may be used as needed as the base resin of the resin composition used to form the light reflection insulating protective film 34. For the base resin of the resin composition that forms the light reflection insulating protective film 34, the same or same-series resin as the resin composition that forms the transparent protective film 35 is preferably used as the base resin. For the transparent protective film 35, an acrylic polyurethane resin is preferably used as a main material resin as will be described later. Thus, when the base resin of the resin composition that forms the transparent protective film 35 is an acrylic polyurethane resin, the base resin of the resin composition for forming the light reflection insulating protective film 34 is more preferably a urethane resin or an acrylic polyurethane resin.

In addition to titanium oxide, at least one selected from among alumina, barium sulfate, magnesia, aluminum nitride, boron nitride, barium titanate, kaolin, talc, calcium carbonate, zinc oxide, silica, mica powder, granulated glass, powdered nickel, and powdered aluminum may be used as the inorganic filler to be contained as a white pigment in the resin composition that forms the light reflection insulating protective film 34.

The thickness of the light reflection insulating protective film 34 is greater than or equal to 5 $\mu$m and more preferably greater than or equal to 7 $\mu$m. The thickness of the light reflection insulating protective film 34 is less than or equal to 50 $\mu$m and more preferably less than or equal to 20 $\mu$m. When the thickness of the light reflection insulating protective film 34 is greater than or equal to 5 $\mu$m, a reduction in the thickness of the light reflection insulating protective film 34 particularly at the edge part of the metal wiring portion 32 can be suppressed. Therefore, the exposure of the metal wiring portion 32 can be suppressed. When the thickness of the light reflection insulating protective film 34 is less than or equal to 50 $\mu$m, for example, the peeling of the light reflection insulating protective film 34 from the metal wiring portion 32 can be suppressed even when the flexible wiring substrate 30 bends during conveyance or the like.

At wavelengths of greater than or equal to 400 nm and less than or equal to 780 nm, the light reflectance of the light reflection insulating protective film 34 is preferably higher than or equal to 65%, more preferably higher than or equal to 70%, and further preferably higher than or equal to 80% in any case. In this case, the LED sheet 20 may, for example, contain 20 parts by mass or more of titanium oxide for 100 parts by mass of urethane or acrylic polyurethane base resin. As a result, the light reflectance of the layer in the case where the thickness of the light reflection insulating protective film 34 is 8 $\mu$m can be set to 75% or higher.

(Transparent Protective Film)

The transparent protective film 35 is formed at the outermost surface of the LED sheet 20 so as to cover the LED chips 21. The transparent protective film 35 has waterproofness and transparency. With the waterproofness of the transparent protective film 35, entry of water into the LED sheet 20 can be suppressed. When the one having a high luminous efficiency, for example, a luminous efficiency of higher than or equal to 150 lm/W, is selected for the LED chips 21, influences in the case of a break of a specific one or some of the LED chips 21 are large in the LED sheet 20. For this reason, making the LED chips 21 hard to break as much as possible is important from the viewpoint of risk management.

The transparent protective film 35 may be formed by using various resin compositions having an acrylic polyurethane resin or the like as a base resin. Other than an acrylic polyurethane resin, a urethane resin, a polyester resin, a phenolic tree, or the like may be used as the base resin of the resin composition used to form the transparent protective film 35. For the base resin of the resin composition that forms the transparent protective film 35, the same or same-series resin as the resin composition that forms the light reflection insulating protective film 34 is preferably used as the base resin. Preferred specific combinations may include a combination of a urethane resin as the base resin of the resin composition that forms the light reflection insulating protective film 34 and an acrylic polyurethane resin as the resin that forms the transparent protective film 35.

The thickness of the transparent protective film 35 is greater than or equal to 10 $\mu$m, preferably greater than or equal to 15 $\mu$m, and more preferably greater than or equal to 20 $\mu$m. The thickness of the transparent protective film 35 is less than or equal to 40 $\mu$m, preferably less than or equal to 30 $\mu$m, and more preferably less than or equal to 25 $\mu$m. By setting the thickness of the transparent protective film 35 within the above range, the good flexibility and thinness, lightweight properties, and good optical characteristics required in emergencies of the LED sheet 20 can be maintained. In addition, sufficient waterproofness required in emergencies can be obtained for the LED sheet 20.

The water resistance of the LED sheet 20 from the transparent protective film 35 is not limited as long as the degradation of the LED chips 21 can be suppressed when water is sprayed onto the LED sheet 20. Such water resistance preferably exhibits IPX4 or higher in the waterproof and dustproof standards stipulated by IEC (International Electrotechnical Commission). Waterproofness higher than or equal to IPX4 is a level to which water splashes from any direction have no harmful effects on the LED chips 21. Specifically, the level is such that water sprayed over all the range of ±180° with respect to the direction normal to the LED sheet 20 for five minutes at a water volume of 10 liters per minute has no harmful effects on the LED chips 21.

(Power Supply Wiring)

As shown in Fig. 8, the power supply wiring 63 is formed on the surface 31a (the surface on the side of the light emitting surface 20a) of the substrate 31. The power supply wiring 63 is a wiring pattern formed on the surface 31a of the substrate 31 from a conductive base material, such as metal foil. The power supply wiring 63 may be formed using the same material and method as the metal wiring portion 32. For example, the power supply wiring 63 may be made of copper foil.

The thickness of the power supply wiring 63 may be set as needed according to, for example, the magnitude of withstand current required from the flexible wiring substrate 30. The thickness T1 (see Fig. 8) of the power supply wiring 63 may be greater than or equal to 18 $\mu$m and is preferably greater than or equal to 35 $\mu$m. The thickness T1 of the power supply wiring 63 may be less than or equal to 100 $\mu$m and is preferably less than or equal to 75 $\mu$m. The line width W1 (see Fig. 8) of the power supply wiring 63 may be greater than or equal to 5 mm and is preferably greater than or equal to 10 mm. Furthermore, the line width W1 of the power supply wiring 63 may be less than or equal to 30 mm and is preferably less than or equal to 20 mm. When the thickness T1 of the power supply wiring 63 is greater than or equal to 18 $\mu$m and the line width W1 of the power supply wiring 63 is greater than or equal to 5 mm, the cross-sectional area of the power supply wiring 63 in a section perpendicular to the longitudinal direction of the power supply wiring 63 can be increased. Thus, the voltage drop can be reduced. When the thickness T1 of the power supply wiring 63 is less than or equal to 100 $\mu$m and the line width W1 of the power supply wiring 63 is less than or equal to 30 mm, sufficient flexibility of the LED sheet 20 can be maintained, and a decrease in handling or the like due to an increase in weight can also be suppressed.

Incidentally, as described above, the LED sheet 20 includes the power receiving terminal 61 and the power feeding terminal 62. Each of the power receiving terminal 61 and the power feeding terminal 62 is connected to the metal wiring portion 32. The power receiving terminal 61 and the power feeding terminal 62 are electric conductors. In other words, the power receiving terminal 61 and the power feeding terminal 62 are terminals formed from a conductive base material, such as metal foil. The power receiving terminal 61 and the power feeding terminal 62 may be formed using the same material and method as the metal wiring portion 32. For example, the power receiving terminal 61 and the power feeding terminal 62 may be made of copper foil. In the present embodiment, the power receiving terminal 61 and the power feeding terminal 62 are formed integrally with the metal wiring portion 32. The power receiving terminal 61 and the power feeding terminal 62 may be formed separately from the metal wiring portion 32.

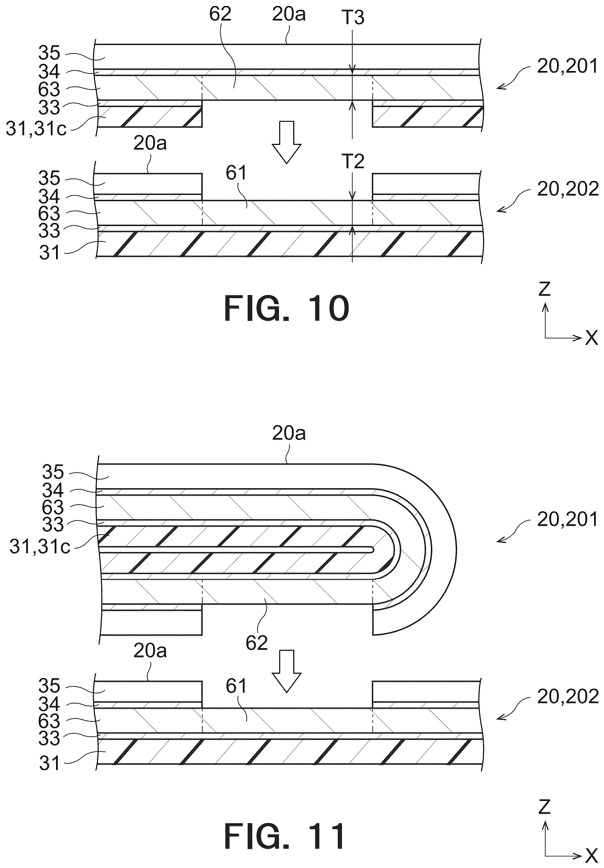

Each of the thickness T2 (see Fig. 10) of the power receiving terminal 61 and the thickness T3 (see Fig. 10) of the power feeding terminal 62 may be greater than or equal to 18 $\mu$m and is preferably greater than or equal to 35 $\mu$m. Each of the thickness T2 of the power receiving terminal 61 and the thickness T3 of the power feeding terminal 62 may be less than or equal to 100 $\mu$m and is preferably less than or equal to 75 $\mu$m. When each of the thickness T2 of the power receiving terminal 61 and the thickness T3 of the power feeding terminal 62 is less than or equal to 100 $\mu$m, the formation of large unevenness on the light emitting surface 20a can be suppressed. When each of the thickness T2 of the power receiving terminal 61 and the thickness T3 of the power feeding terminal 62 is less than or equal to 75 $\mu$m, the formation of large unevenness on the light emitting surface 20a can be further effectively suppressed. Therefore, the LED sheet assembly 20A can be easily wrapped around the culture tube 10. As shown in Fig. 9, in the present embodiment, the power feeding terminal 62 is formed on the protruding portion 31c.

Here, as described above, the LED sheet assembly 20A includes the first LED sheet 201, the second LED sheet 202, and the third LED sheet 203. The power receiving terminal 61 of the first LED sheet 201 is connected to the controller 40 (see Fig. 3). As shown in Fig. 9, the power feeding terminal 62 of the first LED sheet 201 is connected to the power receiving terminal 61 of the second LED sheet 202. Furthermore, the power feeding terminal 62 of the second LED sheet 202 is connected to the power receiving terminal 61 of the third LED sheet 203. In this case, the power feeding terminal 62 of the first LED sheet 201 and the power receiving terminal 61 of the second LED sheet 202 are connected in parallel with each other. The power feeding terminal 62 of the second LED sheet 202 and the power receiving terminal 61 of the third LED sheet 203 are connected in parallel with each other.

At least part of the power receiving terminal 61 and at least part of the power feeding terminal 62 are exposed. In other words, each of the power receiving terminal 61 and the power feeding terminal 62 has a region not covered with an insulator. The power receiving terminal 61 and the power feeding terminal 62 may be connected to each other by bringing the exposed portion of the power receiving terminal 61 and the exposed portion of the power feeding terminal 62 into contact with each other. In an example, the power feeding terminal 62 of the first LED sheet 201 may be connected to the power receiving terminal 61 of the second LED sheet 202 as described below. For example, initially, as shown in Fig. 10, in the first LED sheet 201, the power feeding terminal 62 is exposed by removing part of the protruding portion 31c of the substrate 31 and part of the adhesive layer 33. In the illustrated example, the surface of the power feeding terminal 62, on the side opposite to the light emitting surface 20a, is exposed. In the second LED sheet 202, the power receiving terminal 61 is exposed by removing part of the transparent protective film 35 and part of the light reflection insulating protective film 34. In the illustrated example, the surface of the power receiving terminal 61, on the side of the light emitting surface 20a, is exposed. Then, the power feeding terminal 62 of the first LED sheet 201 and the power receiving terminal 61 of the second LED sheet 202 may be connected to each other by bringing the power feeding terminal 62 and the power receiving terminal 61 into contact with each other. The LED sheet 20 may be manufactured such that the power receiving terminal 61 and the power feeding terminal 62 are exposed in advance. The power feeding terminal 62 may be allowed to be folded back to the back surface 31b of the substrate 31. In this case, for example, initially, as shown in Fig. 11, in the first LED sheet 201, the power feeding terminal 62 may be exposed from part of the transparent protective film 35 and part of the light reflection insulating protective film 34. In the illustrated example, of the surfaces of the power feeding terminal 62, the surface on the side of the light emitting surface 20a is exposed. Then, the power feeding terminal 62 of the first LED sheet 201 and the power receiving terminal 61 of the second LED sheet 202 may be brought into contact with each other by bending the protruding portion 31c of the first LED sheet 201. In these cases, the power receiving terminal 61 and the power feeding terminal 62 may be joined to each other by, for example, solder, conductive paste, or crimping. Although not shown in the drawing, of the surfaces of the power receiving terminal 61, the surface on the side opposite to the light emitting surface 20a may be exposed.

In the LED sheet assembly 20A, the power receiving terminal 61 and the power feeding terminal 62 may be covered with an insulator (for example, the substrate 31, the light reflection insulating protective film 34, or the transparent protective film 35). In this case, for example, as shown in Figs. 10 and 11, the power receiving terminal 61 and the power feeding terminal 62 may be connected to each other by bringing the power receiving terminal 61 and the power feeding terminal 62 into contact with each other, and then the power receiving terminal 61 and the power feeding terminal 62 may be covered with an insulator. Although not shown in the drawing, after the power receiving terminal 61 and the power feeding terminal 62 are connected to each other, the power receiving terminal 61 and the power feeding terminal 62 may be covered with an insulator such as adhesive tape having electrical insulation properties.

As shown in Figs. 1 and 2, in the present embodiment, the LED sheet 20 is adhering to the culture tube 10 by an adhesive member 50. Thus, it is possible to suppress the interposition of an air layer between the LED sheet 20 and the culture tube 10. Here, when an air layer is interposed between the LED sheet 20 and the culture tube 10, the light from the LED chips 21 reflects on the outer surface of the culture tube 10 due to the difference between the refractive index of air and the refractive index of the culture tube 10. Then, there is a possibility that the utilization efficiency of light in the culture system 1 decreases due to an increase in the reflectance of light. In contrast, in the present embodiment, it is possible to suppress the interposition of an air layer between the LED sheet 20 and the culture tube 10. Therefore, the reflection of light from the LED chips 21 on the outer surface of the culture tube 10 can be suppressed. As a result, it is possible to further enhance the utilization efficiency of light. In the present embodiment, the adhesive member 50 is provided so as to cover the entire light emitting surface 20a of the LED sheet 20.

The adhesive member 50 preferably contains ethylene vinyl acetate or polyvinyl butyral. The adhesive member 50 may contain a pressure-sensitive adhesive. This pressure-sensitive adhesive is not limited. As for pressure-sensitive adhesives, for example, natural rubber-based ones, butyl rubber, polyisoprene, polyisobutylene, polychloroprene, styrene polymers, silicone resins, acrylic resins, vinyl acetate resins such as polyvinyl acetate and ethylene-vinyl acetate copolymer, urethane resins, acrylonitrile, hydrocarbon resins, alkylphenol resins, and rosin-based resins such as rosin, rosin triglyceride, and hydrogenated rosin can be applied. The adhesive member 50 may be a commercially available double-sided adhesive film.

The refractive index of the adhesive member 50 may be greater than or equal to 1.48, may be greater than or equal to 1.50, or may be greater than or equal to 1.51. The refractive index of the adhesive member 50 may be less than or equal to 1.60, may be less than or equal to 1.58, or may be less than or equal to 1.56. Here, as described above, for example, glass (refractive index: approximately 1.55) or polycarbonate (refractive index: approximately 1.58) is used for the culture tube 10. For this reason, the refractive index of the adhesive member 50 is greater than or equal to 1.48 and less than or equal to 1.60, so the difference between the refractive index of the adhesive member 50 and the refractive index of the culture tube 10 can be reduced. Thus, the reflection of light from the LED chips 21 on the outer surface of the culture tube 10 can be suppressed. Therefore, it is possible to further enhance the utilization efficiency of light. The refractive indices of the adhesive member 50 and the culture tube 10 can be measured by using, for example, a refractometer (manufactured by Atago Co., Ltd., Abbe refractometer, DR-A1 (product name)).

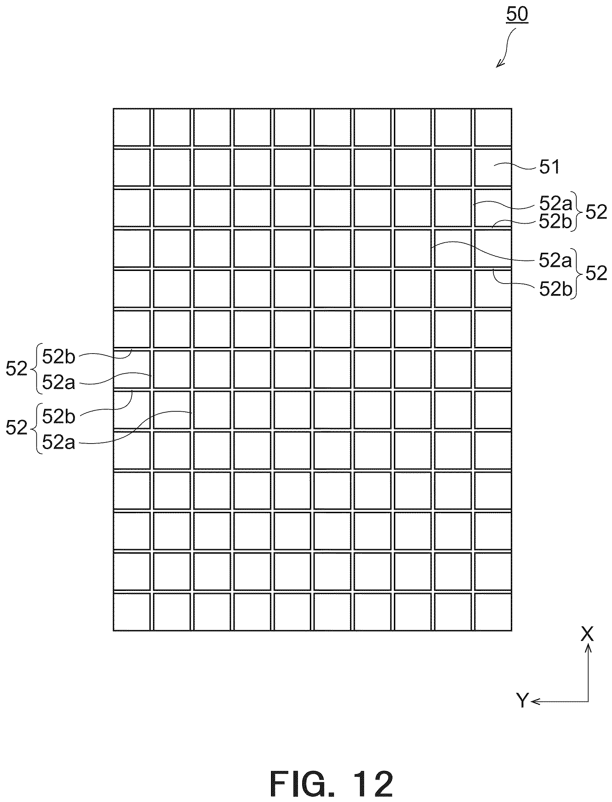

As shown in Fig. 2, the surface 51 of the adhesive member 50 faces the culture tube 10. As shown in Fig. 12, a mesh-like recess 52 is formed on the surface 51. The recess 52 plays a role as an air vent mechanism for releasing air interposed between the adhesive member 50 and the culture tube 10.

In the present embodiment, the recess 52 includes a plurality of first parts 52a and a plurality of second parts 52b. Each first part 52a extends in the first array direction (X direction) of the above-described LED chips 21. In the illustrated example, the intervals between the first parts 52a are equal to each other. The first parts 52a may extend in a direction inclined with respect to the X direction. The intervals between the first parts 52a may be different from each other.

Each second part 52b extends in the second array direction (Y direction) of the above-described LED chips 21. In the illustrated example, the intervals between the second parts 52b are equal to each other. The second parts 52b may extend in a direction inclined with respect to the Y direction. The intervals between the second parts 52b may be different from each other.

The peel strength between the LED sheet 20 and the culture tube 10 is preferably greater than or equal to 3 N/15mm, more preferably greater than or equal to 4 N/15mm, and further preferably greater than or equal to 5 N/15mm. The peel strength between the LED sheet 20 and the culture tube 10 is preferably less than or equal to 30 N/15mm, more preferably less than or equal to 20 N/15mm, and further preferably less than or equal to 15 N/15mm. When the peel strength between the LED sheet 20 and the culture tube 10 is greater than or equal to 3 N/15mm, it is possible to suppress the peeling of the LED sheet 20 from the culture tube 10 during use. When the peel strength between the LED sheet 20 and the culture tube 10 is less than or equal to 30 N/15mm, it is possible to suppress adhesion of, for example, part of the adhesive member 50 to the culture tube 10 when the LED sheet 20 is peeled from the culture tube 10. When the peel strength between the LED sheet 20 and the culture tube 10 is less than or equal to 30 N/15mm, it is easy to peel the LED sheet 20 from the culture tube 10 when the LED sheet 20 is, for example, replaced. The peel strength between the LED sheet 20 and the culture tube 10 can be measured by using, for example, a material testing machine (manufactured by A&D Company, Limited, Tensilon universal testing machine RTF-2325 (product name)).

The photosynthetic photon flux density (PPFD) $I_0$ of the LED sheet 20 is preferably greater than or equal to 250 $\mu$mol/m$^{-2}$·s$^{-1}$ in any region within the culture tube 10. When the photosynthetic photon flux density $I_0$ of the LED sheet 20 is greater than or equal to 250 $\mu$mol/m$^{-2}$·s$^{-1}$, the growth efficiency of algae can be improved. A PPFD can be measured by using a measuring device, such as a quantameter (for example, Quantum sensor LI-190R and Light meter LI-250A manufactured by LI-COR Biosciences, U.S.).

Here, where the molar extinction coefficient of the culture solution CS in the culture tube 10 is $\varepsilon$ (L·cm$^{-1}$·g$^{-1}$), the molarity of the culture solution CS is C (g·L$^{-1}$), and the inside diameter d1 (see Fig. 1) of the culture tube 10 is D (cm),

the photosynthetic photon flux density $I_0$ of the LED sheet 20 preferably satisfies the following relationship.

$$250/10^{(-\varepsilon CD/2)} \leq I_0 \leq 250/10^{(-\varepsilon CD)}$$

Thus, it is possible to improve the growth efficiency of algae and reduce power consumption.

By the way, where absorbance is denoted by A and transmittance is denoted by T, the following equation (1) holds true according to the Lambert-Beer law.

$$A = -\log_{10}T = \varepsilon CD \qquad (1)$$

Where the photosynthetic photon flux density that has passed through the culture solution CS is denoted by I, the transmittance T can be expressed by the following equation (2).

$$T = I/I_0 \qquad (2)$$

Therefore, using the equations (1) and (2), the photosynthetic photon flux density $I_0$ can be expressed by the following equation (3).

$$I_0 = I/10^{-\varepsilon CD} \qquad (3)$$

As described above, the photosynthetic photon flux density $I_0$ of the LED sheet 20 is preferably greater than or equal to 250 $\mu$mol/m$^{-2}$·s$^{-1}$ in any region within the culture tube 10. It is believed that the position where the photosynthetic photon flux density $I_0$ of the LED sheet 20 is lowest within the culture tube 10 is at the radial center of the culture tube 10. Therefore, the photosynthetic photon flux density $I_0$ of the LED sheet 20 is preferably greater than or equal to 250 $\mu$mol/m$^{-2}$·s$^{-1}$ at the radial center of the culture tube 10. In this case, when the relationship

$$250/10^{(-\varepsilon CD/2)} \leq I_0$$

is satisfied, the photosynthetic photon flux density $I_0$ of the LED sheet 20 can be set to 250 $\mu$mol/m$^{-2}$·s$^{-1}$ or greater at the radial center of the culture tube 10. Therefore, when the relationship $2507/10^{(\varepsilon CD/2)} \leq I_0$ is satisfied, it is possible to improve the growth efficiency of algae. When the relationship $I_0 \leq 250/10^{(-\varepsilon CD)}$ is satisfied, power consumption can be reduced.

(Manufacturing Method for LED Sheet)

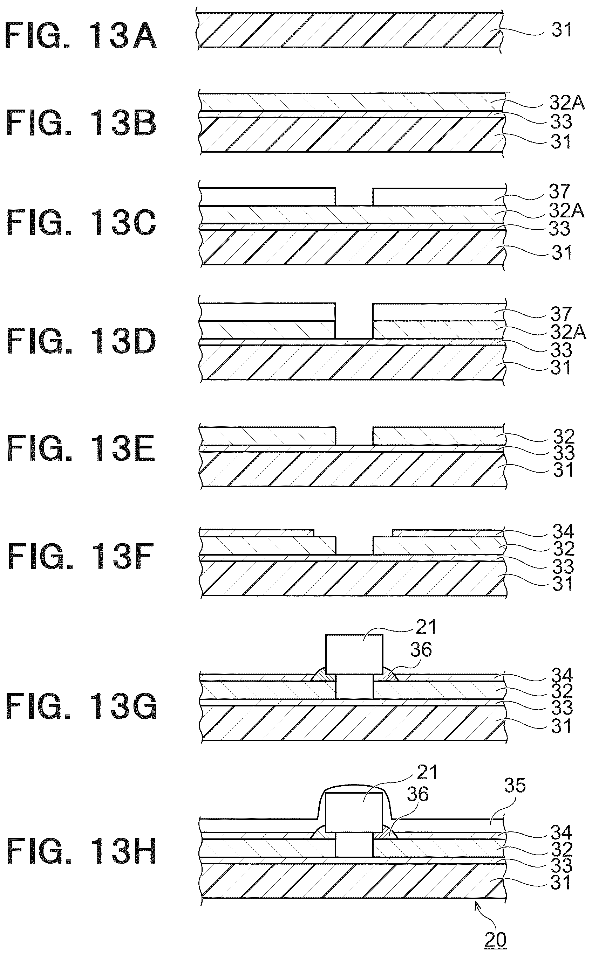

Next, a manufacturing method for the LED sheet 20 according to the present embodiment will be described with reference to Figs. 13A to 13H.

Initially, a substrate 31 is prepared (Fig. 13A). Subsequently, metal foil 32A, such as copper foil, which is the material of a metal wiring portion 32, a power receiving terminal 61, a power feeding terminal 62, and a power supply wiring 63 (hereinafter, simply referred to as a metal wiring portion 32 and the like), is laminated on the surface 31a (see Fig. 7) of the substrate 31 (Fig. 13B). The metal foil 32A is bonded to the surface 31a of the substrate 31 by an adhesive layer 33 of, for example, a urethane-based adhesive or the like. Alternatively, the metal foil 32A may be directly formed on the surface 31a of the substrate 31 by electrolytic plating or vapor deposition (sputtering, ion plating, electron-beam evaporation, vacuum evaporation, chemical vapor deposition, or the like). Alternatively, the substrate 31 may be directly welded onto the metal foil 32A.

After that, an etching mask 37 patterned in a shape required for the metal wiring portion 32 and the like is formed on the surface of the metal foil 32A (Fig. 13C). The etching mask 37 is provided such that a part corresponding to the wiring pattern of the metal foil 32A, which becomes the metal wiring portion 32 and the like, does not corrode due to etchant. A method of forming the etching mask 37 is not limited. The etching mask 37 may be formed by, for example, exposing a photoresist or a dry film to light through a photomask and then developing the photoresist or the dry film. The etching mask 37 may be formed on the surface of the metal foil 32A by a printing technology such as an ink-jet printer.

Subsequently, the metal foil 32A at positions not covered with the etching mask 37 is removed by immersion liquid (Fig. 13D). As a result, a part of the metal foil 32A, other than the part to be the metal wiring portion 32 and the like, is removed.

After that, the etching mask 37 is removed by using alkaline stripper. As a result, the etching mask 37 is removed from the surface of the metal wiring portion 32 and the like (Fig. 13E).

Subsequently, a light reflection insulating protective film 34 is laminated on the metal wiring portion 32 and the like (Fig. 13F). Formation of the light reflection insulating protective film 34 is not limited as long as a coating method is capable of uniformly coating a material resin composition that makes up the light reflection insulating protective film 34, and, for example, a method, such as screen printing, offset printing, dip coater, and brush coating, can be used. Alternatively, a light reflection insulating protective film 34 may be formed by coating an insulating protective film material having photosensitivity over the entire surface, exposing only necessary portions to light through a photomask, and then developing. The light reflection insulating protective film 34 does not need to be formed on the power feeding terminal 62 and the power supply wiring 63.

After that, LED chips 21 are mounted on the metal wiring portion 32 (Fig. 13G). In Fig. 13G and Fig.

13H (described later), regulators 45 and the like are not shown for clear illustration. In this case, LED chips 21 are joined to the metal wiring portion 32 via solder portions 36 by soldering. Joining by soldering may be performed by a reflow method or a laser method or may be joining with a conductive resin.

Subsequently, a transparent protective film 35 is formed so as to cover the light reflection insulating protective film 34 and the LED chips 21 (Fig. 13H). The transparent protective film 35 is preferably formed by a formation method by spraying a transparent resin composition by spraying (hereinafter, referred to as spray coating) or a formation method by curtain coating. Formation of the transparent protective film 35 by spray coating can be performed in a manner such that, for example, a coating film is formed by spraying coating liquid containing acrylic polyurethane resin to a desired region on the flexible wiring substrate 30 with a spray coater. Formation of the transparent protective film 35 by curtain coating can be performed in a manner such that, for example, a coating film is formed by dripping curtain coating liquid containing acrylic polyurethane resin to a desired region on the flexible wiring substrate 30 with a curtain coater.

Not limited to the above-described methods, the LED sheet 20 according to the present embodiment can be manufactured by known methods of manufacturing an existing known flexible wiring substrate for LED chips or various LED sheets formed by mounting LED chips on a flexible wiring substrate.

(Culture Factory)

Fig. 14 is a view schematically showing the configuration of a culture factory 90 using the culture system 1 according to the present embodiment. The culture factory 90 includes a building 91 and a plurality of culture racks 80 placed in the building 91.

As shown in Fig. 15, the culture rack 80 includes a plurality of (four) posts 82, and a pair of rack boards 81 arranged in an up and down direction with a space along the posts 82. A plurality of culture systems 1 is placed between the pair of rack boards 81. In the illustrated example, the culture systems 1 are placed such that the longitudinal directions of the culture tubes 10 are parallel to the up and down direction (vertical direction). Although not shown in the drawing, the culture systems 1 may be placed such that the longitudinal directions of the culture tubes 10 are parallel to a horizontal direction.

Here, the controller 40 is placed at a location sufficiently away from the culture tubes 10. For this reason, there are small concerns about variation in the growth of algae due to heat from the controller 40 between culture tubes 10 closer to the controller 40 and culture tubes 10 far from the controller 40.

The LED sheet 20 according to the present embodiment is made thinner than an existing straight-tube lighting unit. Thus, the intervals between the culture systems 1 can be narrowed, so the number of culture systems 1 placed between the pair of rack boards 81 can be increased. As a result, the growth rate of algae per unit area can be increased.

In this way, according to the present embodiment, the LED sheet 20 includes the substrate 31, the metal wiring portion 32, the plurality of LED chips 21 mounted on the metal wiring portion 32, the power receiving terminal 61 connected to the metal wiring portion 32 and used for connection with another LED sheet 20, and the power feeding terminal 62 connected to the metal wiring portion 32 and used for connection with another LED sheet 20. With this configuration, the LED sheets 20 can be easily connected to each other.

Here, the LED sheet 20 has, for example, a size of about 600 mm × 500 mm. Therefore, when a plurality of LED sheets 20 is assembled in the culture factory 90, there is a possibility that assembling is difficult. In contrast, in the present embodiment, the LED sheets 20 can be easily connected to each other. Therefore, a plurality of LED sheets 20 can be easily assembled in the culture factory 90. It is also easy to deliver the LED sheet assembly 20A, in which the LED sheets 20 are connected, to the culture factory 90.

Since the LED sheets 20 can be easily connected to each other, replacement of the LED sheets 20 can be easily performed. In this case, when the LED chips 21 break in a predetermined LED sheet 20, only that LED sheet 20 can be easily replaced, so the LED sheet assembly 20A can be easily repaired.

According to the present embodiment, the substrate 31 includes the protruding portion 31c that protrudes outward. The power feeding terminal 62 is formed on the protruding portion 31c. Thus, the power feeding terminal 62 can be easily connected to the power receiving terminal 61 of another LED sheet 20.

According to the present embodiment, the LED sheet 20 includes the indicating part 25 that indicates the ON state of the LED chips 21 and the OFF state of the LED chips 21. Thus, even when the LED sheets 20 are wrapped around the culture tube 10, it is possible to easily check whether the LED chips 21 are in the ON state or in the OFF state.

According to the present embodiment, the substrate 31 has flexibility. In this way, since the LED sheet 20 is a sheet-like LED lighting unit having the flexible substrate 31, the weight of the LED sheet assembly 20A and the culture system 1 can be reduced. The LED sheet 20 of the culture system 1 according to the present embodiment can reduce the overall thickness compared to a straight-tube LED bar light including an array of a plurality of LEDs. Therefore, when the LED sheet 20 is wrapped around the culture tube 10, the bulk of the culture system 1 can be suppressed. Thus, installation space for the culture system 1 can be reduced. Since the LED sheet 20 is a sheet-like LED lighting unit having the flexible substrate 31, the LED sheet 20 can be easily attached to culture tubes 10 of various shapes.

According to the present embodiment, in the LED sheet assembly 20A, the power feeding terminal 62 of the first LED sheet 201 and the power receiving terminal 61 of the second LED sheet 202 are connected in parallel with each other. Even when one of the LED sheets 20 breaks, it is possible to suppress influences on the other one of the LED sheets 20. Thus, it is possible to suppress an extreme decrease in the illuminance of the overall LED sheet assembly 20A.

According to the present embodiment, the LED sheet assembly 20A further includes the connecting member 26 attached to the first LED sheet 201 and the second LED sheet 202. The second LED sheet 202 is connected to the first LED sheet 201 via the connecting member 26. Thus, the LED sheets 20 can be further easily connected to each other, and it is possible to suppress unintentional detachment of the second LED sheet 202 from the first LED sheet 201.

Furthermore, according to the present embodiment, the culture system 1 includes the culture tube 10 that cultures algae and the LED sheet assembly 20A that covers the outer surface of the culture tube 10. Thus, it is possible to suppress leakage of light, applied from the LED sheet 20 to the culture tube 10, to the outside of the culture tube 10. Therefore, it is possible to enhance the utilization efficiency of light. Since the LED sheet 20 covers the outer surface of the culture tube 10, it is also possible to apply light from all around the trunk portion 11 into the culture tube 10. Therefore, it is possible to enhance the growth efficiency of algae.

[Modifications]

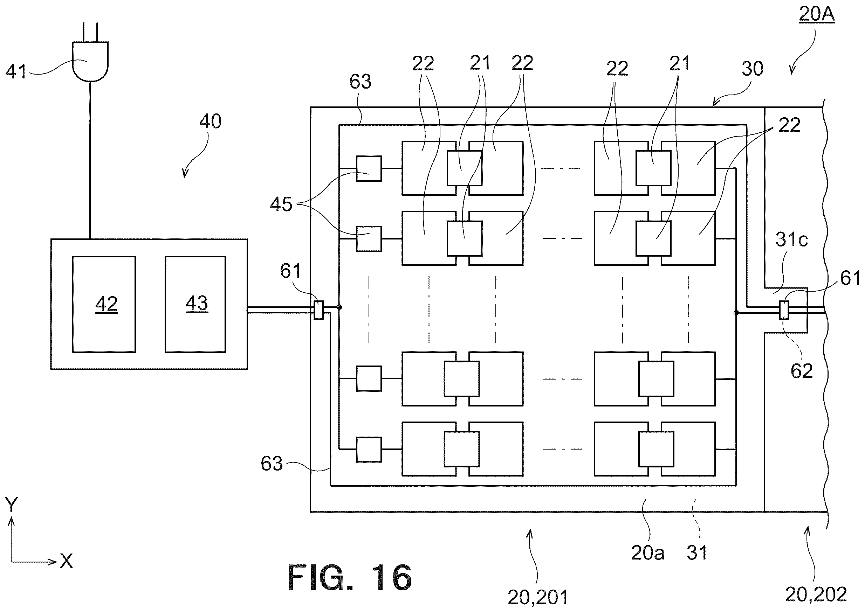

Next, modifications of the LED sheet assembly 20A and the culture system 1 according to the present embodiment will be described with reference to Figs. 16 to 24. In Figs. 16 to 24, like reference signs are assigned to the same portions in Figs. 1 to 15, and the detailed description is omitted.

(First Modification)

In Fig. 16, the power receiving terminal 61 of the second LED sheet 202 is formed on the protruding portion 31c of the second LED sheet 202. In the illustrated example, the first LED sheet 201 has no protruding portion 31c, and the power feeding terminal 62 of the first LED sheet 201 is not formed on the protruding portion 31c. However, the configuration is not limited thereto. The first LED sheet 201 may have a protruding portion 31c, and the power feeding terminal 62 of the first LED sheet 201 may be formed on the protruding portion 31c of the first LED sheet 201. In the present modification as well, since the power receiving terminal 61 is formed on the protruding portion 31c, the power receiving terminal 61 can be easily connected to the power feeding terminal 62 of another LED sheet 20.

(Second Modification)

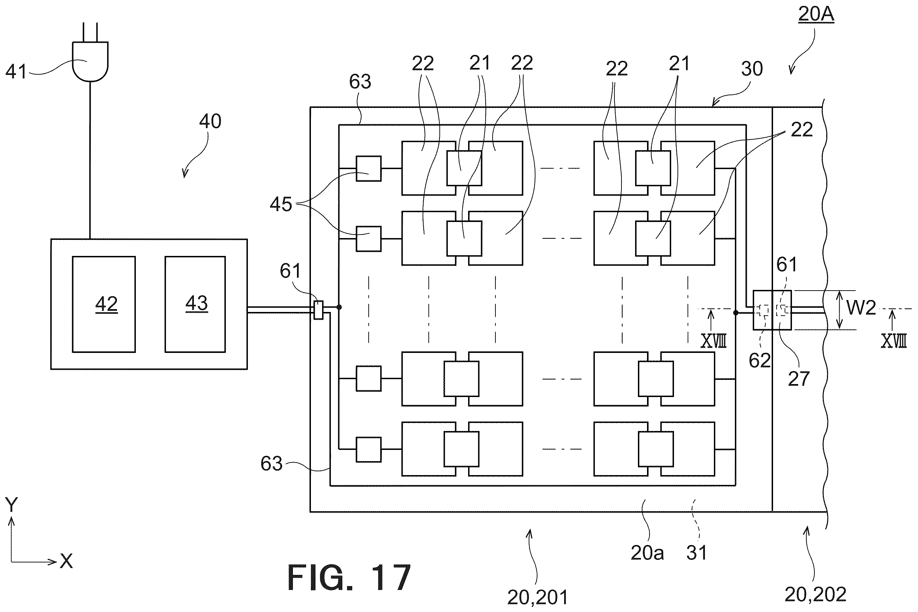

In Fig. 17, the LED sheet assembly 20A further includes a connecting member 27. In the illustrated example, the connecting member 27 is detachably attached to the first LED sheet 201 and the second LED sheet 202. The power feeding terminal 62 of the first LED sheet 201 and the power receiving terminal 61 of the second LED sheet 202 are connected to each other via the connecting member 27. Although not shown in the drawing, the LED sheet assembly 20A may include a plurality of the connecting members 27, and the connecting member 27 may be detachably attached to the second LED sheet 202 and the third LED sheet 203. The power feeding terminal 62 of the second LED sheet 202 and the power receiving terminal 61 of the third LED sheet 203 may be connected to each other via the connecting member 27. The connecting member 27 may be attached to the first LED sheet 201 or the like in a manner such that the connecting member 27 cannot be detached from the first LED sheet 201 or the like. In this case, the connecting member 27 may be attached to the first LED sheet 201 or the like by using solder, adhesive, or the like.

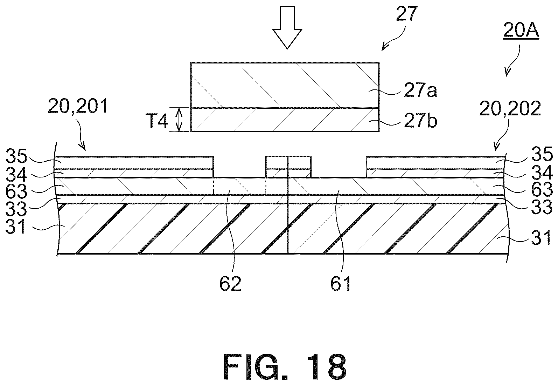

As shown in Fig. 18, the connecting member 27 includes an insulating layer 27a and a metal layer 27b. Of these, the insulating layer 27a may be formed using the same material and method as the light reflection insulating protective film 34. The metal layer 27b may be formed using the same material and method as the metal wiring portion 32. For example, the metal layer 27b may be made of copper foil. The thickness T4 of the metal layer 27b may be greater than or equal to 18 $\mu$m and is preferably greater than or equal to 35 $\mu$m. The thickness T4 of the metal layer 27b may be less than or equal to 100 $\mu$m and is preferably less than or equal to 75 $\mu$m. The width W2 (the length in the Y direction, see Fig. 17) of the metal layer 27b may be greater than or equal to 5 mm and is preferably greater than or equal to 10 mm. Furthermore, the width W2 of the metal layer 27b may be less than or equal to 30 mm and is preferably less than or equal to 20 mm. When the thickness T4 of the metal layer 27b is greater than or equal to 18 $\mu$m and the width W2 of the metal layer 27b is greater than or equal to 5 mm, the cross-sectional area of the metal layer 27b in a section perpendicular to the longitudinal direction (X direction) of the metal layer 27b can be increased. Thus, the voltage drop can be reduced. When the thickness T4 of the metal layer 27b is less than or equal to 100 $\mu$m and the width W2 of the metal layer 27b is less than or equal to 30 mm, sufficient flexibility of the LED sheet assembly 20A can be maintained, and a decrease in handling due to an increase in weight can also be suppressed.

In the illustrated example, the connecting member 27 connects the power receiving terminal 61 and the power feeding terminal 62 on the side of the light emitting surface 20a of the LED sheet 20; however, the configuration is not limited thereto. The connecting member 27 may connect the power receiving terminal 61 and the power feeding terminal 62 on the side opposite to the light emitting surface 20a of the LED sheet 20.

According to the present modification, the LED sheet assembly 20A further includes the connecting member 27 detachably attached to the first LED sheet 201 and the second LED sheet 202. The power feeding terminal 62 of the first LED sheet 201 and the power receiving terminal 61 of the second LED sheet 202 are connected to each other via the connecting member 27. In this case as well, the power feeding terminal 62 can be easily connected to the power receiving terminal 61 of another LED sheet 20. Therefore, the LED sheets 20 can be easily connected to each other.

(Third Modification)

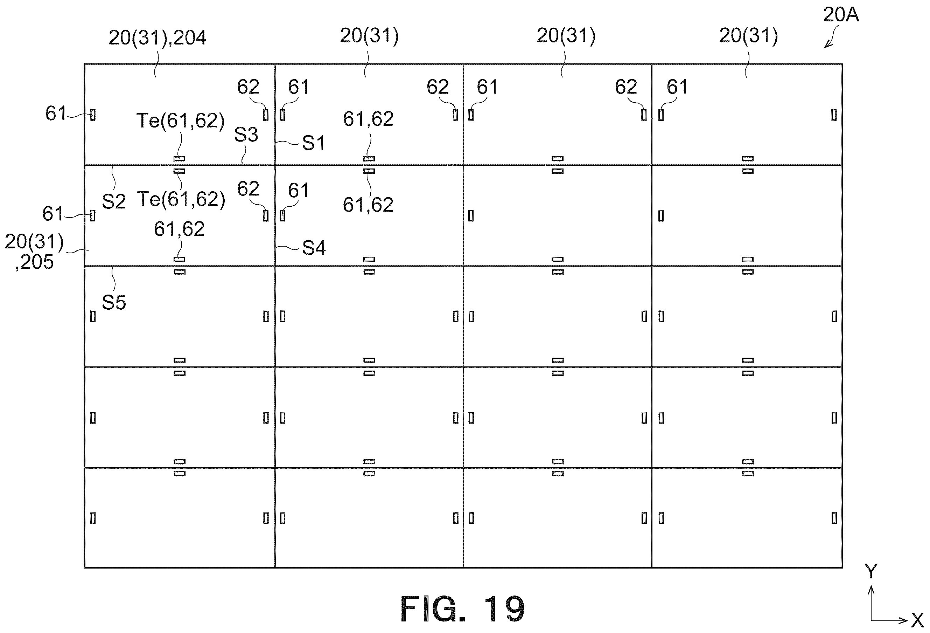

In Fig. 19, a plurality of LED sheets 20 is arranged in the first array direction (X direction) and the second array direction (Y direction) of the LED chips 21. Specifically, the LED sheets 20 are arranged in multiple rows and multiple columns in plan view.

For example, in Fig. 19, four LED sheets 20 are arranged in the first array direction (X direction) of the LED chips 21. Furthermore, five rows of four LED sheets are arranged in the second array direction (Y direction) of the LED chips 21. The number of LED sheets 20 arranged is not limited thereto.

Here, when a plurality of LED sheets 20 is arranged in the first array direction (X direction) and the second array direction (Y direction) of the LED chips 21, one LED sheet 20 may include a plurality of power feeding terminals 62. In other words, one LED sheet 20 may supply electric power to another LED sheet 20 adjacent in the X direction and supply electric power to another LED sheet 20 adjacent in the Y direction.

In this case, when the planar shape of the substrate 31 is a k-sided polygon (where k is a natural number greater than or equal to three), a plurality of the power feeding terminals 62 is provided in a predetermined LED sheet 20. In the predetermined LED sheet 20, the power feeding terminals 62 may be provided at positions respectively corresponding to at least two sides among k sides of the k-sided polygon. For example, in the example shown in Fig. 19, in the LED sheet 204, the power feeding terminals 62 may be provided at positions respectively corresponding to two sides among the four sides of the quadrilateral. In other words, in the LED sheet 204, the power feeding terminals 62 are provided at positions respectively corresponding to a side s1 and a side s2 among the four sides. In the LED sheet 205, the power feeding terminals 62 are provided at positions respectively corresponding to three sides among the four sides of the quadrilateral. In other words, in the LED sheet 205, the power feeding terminals 62 are provided at positions respectively corresponding to a side s3, a side s4, and a side s5 among the four sides. When, for example, the LED sheet 204 supplies electric power to the LED sheet 205 adjacent in the Y direction in Fig. 19, a terminal Te provided near the LED sheet 205 among the terminals of the LED sheet 204 can be provided as the power feeding terminal 62. On the other hand, when the LED sheet 204 supplies electric power to the LED sheet 205 adjacent in the Y direction, the terminal Te of the LED sheet 204 can be provided as the power feeding terminal 62.

For example, in a predetermined LED sheet 20, k-1 or less power feeding terminals 62 may be provided. The power feeding terminals 62 may be provided at positions respectively corresponding to sides different from each other among k sides of the k-sided polygon. For example, in the example shown in Fig. 19, in the LED sheet 204, two power feeding terminals 62 are provided. The power feeding terminals 62 are provided at positions respectively corresponding to sides s1, s2 different from each other among four sides of the quadrilateral. In the LED sheet 205, three power feeding terminals 62 are provided. The power feeding terminals 62 are provided at positions respectively corresponding to sides s3, s4, s5 different from one another among four sides of the quadrilateral. Although not shown in the drawing, a plurality of power feeding terminals 62 may be provided at a position corresponding to one side.

According to the present modification, the power feeding terminals 62 are provided at positions respectively corresponding to at least two sides among the k sides of the k-sided polygon. Thus, a plurality of LED sheets 20 can be easily connected to each other, so the size of the LED sheet assembly 20A can be increased.

According to the present modification, k-1 or less power feeding terminals 62 are provided, and the power feeding terminals 62 are provided at positions respectively corresponding to sides different from each other among k sides of the k-sided polygon. In this case as well, a plurality of LED sheets 20 can be easily connected to each other, so the size of the LED sheet assembly 20A can be increased.

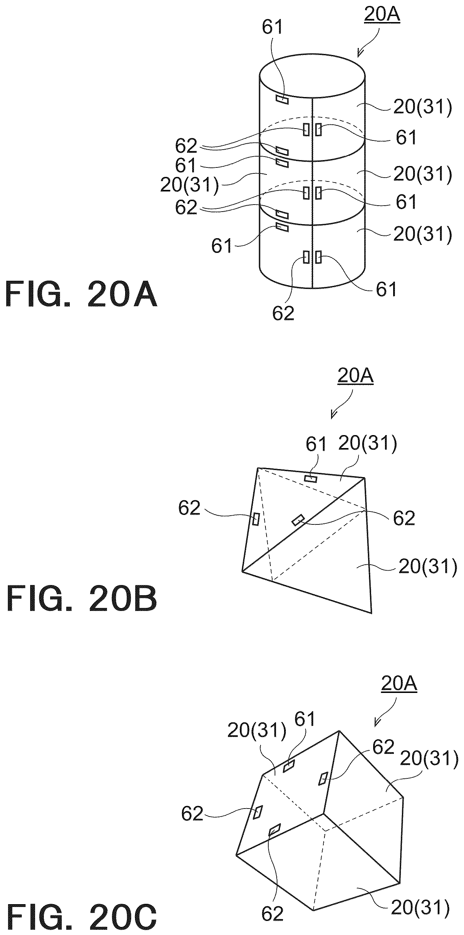

As described above, when the planar shape of the substrate 31 is a k-sided polygon, k-1 or less power feeding terminals 62 may be provided in each of the LED sheets 20. For example, in this case, as shown in Fig. 20A, when the planar shape of the substrate 31 is quadrilateral, two power feeding terminals 62 may be provided in a predetermined LED sheet 20. In this case, a plurality of LED sheets 20 may be connected to each other such that the LED sheet assembly 20A becomes a cylindrical shape as a whole.

As shown in Fig. 20B, when the planar shape of the substrate 31 is triangular, two power feeding terminals 62 may be provided in a predetermined LED sheet 20. In this case, a plurality of LED sheets 20 may be connected to each other such that the LED sheet assembly 20A constitutes an octahedron as a whole.

As shown in Fig. 20C, when the planar shape of the substrate 31 is quadrilateral, three power feeding terminals 62 may be provided in a predetermined LED sheet 20. In this case, a plurality of LED sheets 20 may be connected to each other such that the LED sheet assembly 20A constitutes a hexahedron as a whole.

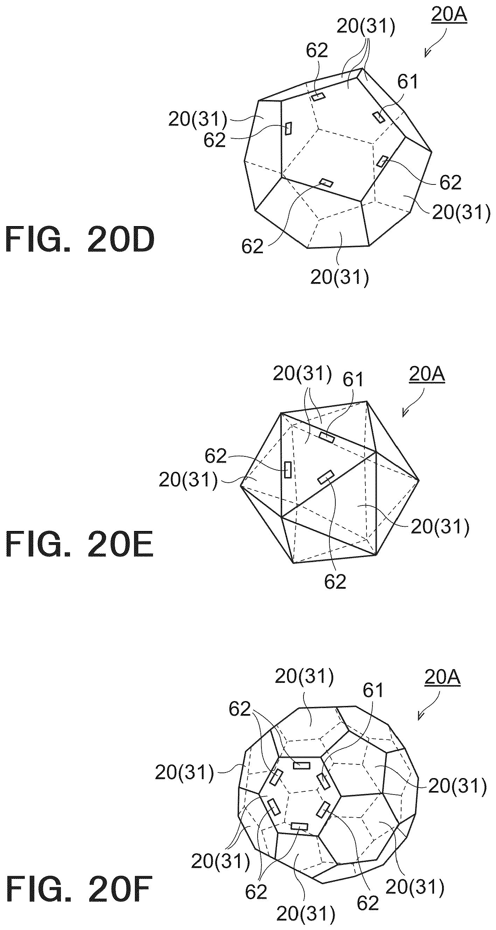

As shown in Fig. 20D, when the planar shape of the substrate 31 is pentagonal, four power feeding terminals 62 may be provided in a predetermined LED sheet 20. In this case, a plurality of LED sheets 20 may be connected to each other such that the LED sheet assembly 20A constitutes a dodecahedron as a whole.

As shown in Fig. 20E, when the planar shape of the substrate 31 is triangular, two power feeding terminals 62 may be provided in a predetermined LED sheet 20. In this case, a plurality of LED sheets 20 may be connected to each other such that the LED sheet assembly 20A constitutes an icosahedron as a whole.

Furthermore, as shown in Fig. 20F, when the planar shape of the substrate 31 is hexagonal or pentagonal, five power feeding terminals 62 may be provided in a predetermined LED sheet 20 where the planar shape of the substrate 31 is hexagonal. In this case, a plurality of LED sheets 20 may be connected to each other such that the LED sheet assembly 20A constitutes a truncated icosahedron as a whole.

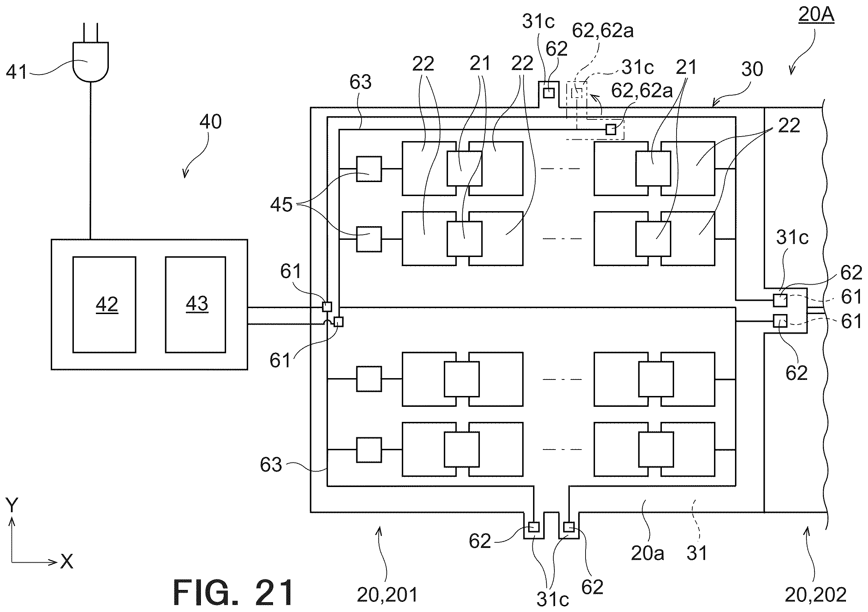

As shown in Fig. 21, when one LED sheet 20 supplies electric power to another LED sheet 20 adjacent in the X direction and supplies electric power to another LED sheet 20 adjacent in the Y direction, power feeding terminals 62 may be provided near all the sides of the substrate 31. In this case, protruding portions 31c may be provided at all the sides of the substrate 31. The protruding portion 31c may be formed by creating, for example, a cutout having a C-shape or the like in the substrate 31 and folding back the power supply wiring 63 along with the substrate 31. In the illustrated example, of the power feeding terminals 62, the power feeding terminal 62a is formed on the protruding portion 31c formed by folding back the substrate 31. At this time, the power feeding terminal 62a may be folded back to the surface 31a of the substrate 31 or may be folded back to the back surface 31b of the substrate 31. In this way, the power feeding terminal 62 (and the power receiving terminal 61) may be allowed to be folded back to the surface 31a or the back surface 31b of the substrate 31.

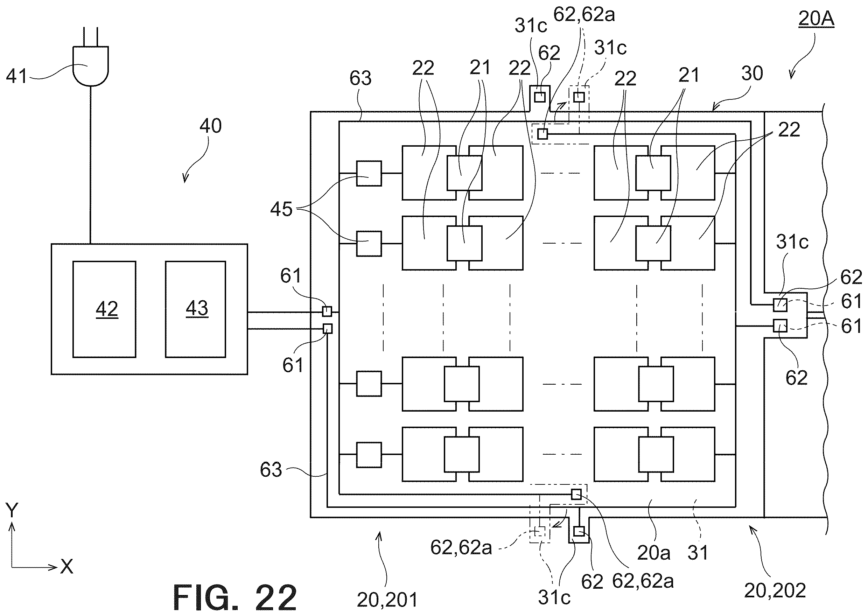

In the example shown in Fig. 21, only the positive (or negative) power feeding terminal 62 is formed on the protruding portion 31c formed by folding back the substrate 31; however, the configuration is not limited thereto. For example, as shown in Fig. 22, the positive power feeding terminal 62 and the negative power feeding terminal 62 may be formed on the protruding portion 31c formed by folding back the substrate 31.

(Fourth Modification)

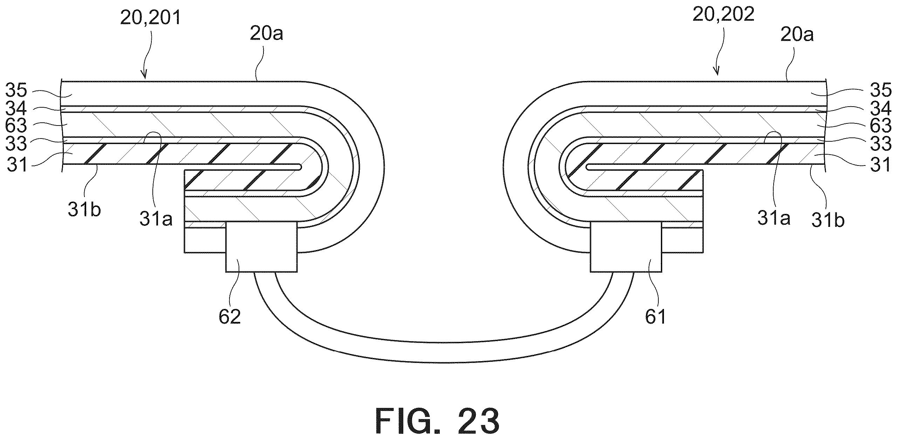

In Fig. 23, the power receiving terminal 61 and the power feeding terminal 62 are allowed to be folded back to the back surface 31b of the substrate 31. In the example shown in Fig. 23, the power receiving terminal 61 and the power feeding terminal 62 are connectors. In this case, a cutout may be formed in the substrate 31, and the power receiving terminal 61 or the power feeding terminal 62 may be folded back along with the substrate 31. As a result, even when the power receiving terminal 61 and the power feeding terminal 62 are composed of thick components, such as connectors, the formation of large unevenness on the light emitting surface 20a can be suppressed. Therefore, the LED sheet assembly 20A can be easily wrapped around the culture tube 10. In this case, the power receiving terminal 61 and the power feeding terminal 62 may be allowed to be folded back to the surface 31a of the substrate 31. As a result, even when the power receiving terminal 61 and the power feeding terminal 62 are composed of thick components, such as connectors, it is possible to suppress the formation of large unevenness on the surface opposite to the light emitting surface 20a.

(Fifth Modification)

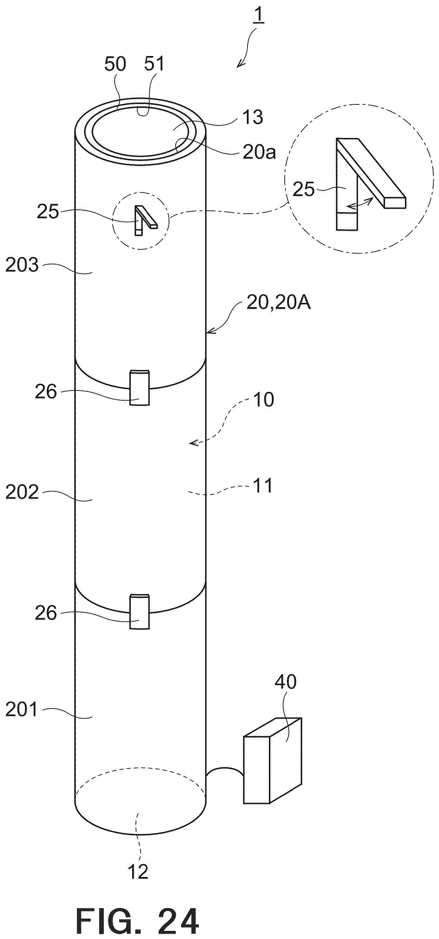

In Fig. 24, the indicating part 25 is configured to be openable and closable. In other words, the indicating part 25 may be formed by forming a C-shaped cutout in the LED sheet 20 and bending the portion where the cutout is formed to expose the indicating part 25. Thus, since the indicating part 25 is usually covered by the LED sheet 20, it is possible to suppress leakage of the light, applied from the LED sheet 20 to the culture tube 10, to the outside of the culture tube 10. Therefore, it is possible to enhance the utilization efficiency of light. The shape of the cutout is not limited to a C-shape and may be a U-shape, an L-shape, a V-shape, or the like.

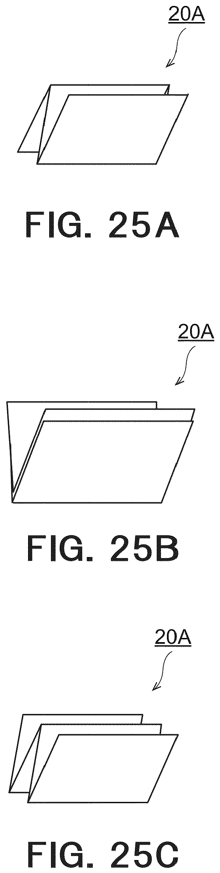

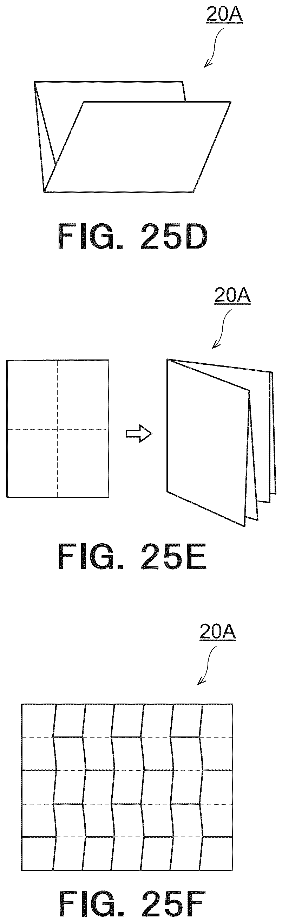

As described above, the LED sheet assembly 20A described in the embodiment and the modifications is foldable. At this time, for example, as shown in Fig. 25A, the LED sheet assembly 20A may be folded in a so-called Z-fold. Alternatively, as shown in Fig. 25B, the LED sheet assembly 20A may be folded in a so-called double parallel fold. Alternatively, as shown in Fig. 25C, the LED sheet assembly 20A may be folded in a so-called 8-page accordion fold (W-fold). Alternatively, as shown in Fig. 25D, the LED sheet assembly 20A may be folded in a so-called letter fold. Alternatively, as shown in Fig. 25E, the LED sheet assembly 20A may be folded in a so-called square fold. Furthermore, as shown in Fig. 25F, the LED sheet assembly 20A may be folded in a so-called Miura fold (registered trademark) in which the LED sheet assembly 20A is folded along zigzag mountain folds (see continuous line) and valley folds (see dashed line). The LED sheet assembly 20A may be folded along the connecting portion between the LED sheets 20 or may be folded such that creases are formed at portions other than the connecting portion between the LED sheets 20.

In the above-described embodiment, the example in which the LED sheet assembly 20A is used in the culture system 1 that cultures algae has been described; however, the configuration is not limited thereto. For example, although not shown in the drawing, the LED sheet assembly 20A may be used in plant and animal growth factories to grow plants and/or animals using artificial light.

The plurality of component elements described in the embodiment and the modifications may be combined as needed. Alternatively, some component elements may be deleted from all the component elements described in the embodiment and the modifications.

**Claims**

1.  An LED sheet comprising:

    a substrate having a first surface and a second surface positioned on a side opposite to the first surface;
    a metal wiring portion positioned on the first surface of the substrate;
    a plurality of LED chips mounted on the metal wiring portion;
    a power receiving terminal connected to the metal wiring portion and used for connection with another LED sheet; and
    a power feeding terminal connected to the metal wiring portion and used for connection with another LED sheet.

2.  The LED sheet according to claim 1, further comprising a power supply wiring that connects the metal wiring portion to the power receiving terminal and the power feeding terminal.

3.  The LED sheet according to claim 2, wherein a thickness of the power supply wiring is greater than or equal to 18 $\mu$m and less than or equal to 100 $\mu$m, and a line width of the power supply wiring is greater than or equal to 5 mm and less than or equal to 30 mm.

4.  The LED sheet according to claim 1, wherein the power receiving terminal and the power feeding terminal are electric conductors, and at least part of the power receiving terminal and at least part of the power feeding terminal are exposed.

5.  The LED sheet according to claim 1, wherein the power receiving terminal and the power feeding terminal are allowed to be folded back to the first surface side or the second surface side.

6.  The LED sheet according to claim 1, wherein the substrate includes a protruding portion that protrudes outward, and at least one of the power receiving terminal and the power feeding terminal is formed on the protruding portion.

7.  The LED sheet according to claim 1, further comprising an indicating part that indicates an ON state of the LED chips and an OFF state of the LED chips.

8. The LED sheet according to claim 1, wherein the substrate has flexibility.

9. The LED sheet according to claim 1, wherein

a planar shape of the substrate is a k-sided polygon (where k is a natural number greater than or equal to three),
a plurality of the power feeding terminals is provided, and
the power feeding terminals are provided at positions respectively corresponding to at least two sides among k sides of the k-sided polygon.

10. The LED sheet according to claim 9, wherein

k-1 or less number of the power feeding terminals are provided, and
the power feeding terminals are provided at positions respectively corresponding to sides different from each other among the k sides of the k-sided polygon.

11. An LED sheet assembly comprising:

a first LED sheet; and
a second LED sheet connected to the first LED sheet, wherein
each of the first LED sheet and the second LED sheet is the LED sheet according to any one of claims 1 to 10, and
the power feeding terminal of the first LED sheet is connected to the power receiving terminal of the second LED sheet.

12. The LED sheet assembly according to claim 11, wherein the power feeding terminal of the first LED sheet and the power receiving terminal of the second LED sheet are connected in parallel with each other.

13. The LED sheet assembly according to claim 11, further comprising

a connecting member attached to the first LED sheet and the second LED sheet, wherein
the power feeding terminal of the first LED sheet and the power receiving terminal of the second LED sheet are connected to each other via the connecting member.

14. The LED sheet assembly according to claim 11, further comprising

a connecting member attached to the first LED sheet and the second LED sheet, wherein

the second LED sheet is connected to the first LED sheet via the connecting member.

15. The LED sheet assembly according to claim 11, wherein the LED sheet assembly is foldable.

16. The LED sheet assembly according to claim 11, wherein the power receiving terminal and the power feeding terminal are covered with an insulator.

17. A culture system comprising:

a culture tube that cultures algae; and
the LED sheet assembly according to claim 11, covering an outer surface of the culture tube.

FIG. 1

1

13

25

10

CS
203

11

20A,20

26

20a

50

51

202

d1

26

201

40

12

# FIG. 2

FIG. 3

EP 4 645 397 A1

Ly

Py

20

22

21

22

21

30

Lx

Px

VII  VII

45

R

20a

X

Y

**FIG. 4**

**FIG. 5A**

**FIG. 5B**

FIG. 6A

FIG. 6B

34

22,22A    36    20a    21    36    22,22B    35

20

32
30 { 33
31

31b    31a

Z
└→X

**FIG. 7**

34

33
30 { 31

31b    31a    63

W1

T1    35

20

Z
└→X

**FIG. 8**

FIG. 9

35
34
62    20a    T3    20,201
63
33
31,31c
20a

35
34    61    T2    20,202
63
33
31

**FIG. 10**

Z
X

20a

35
34
63
33
31,31c
20a

62

35
34    61    20,201
63
33
31    20,202

20a

**FIG. 11**

Z
X

FIG. 12

**FIG. 13A** — 31

**FIG. 13B** — 32A, 33, 31

**FIG. 13C** — 37, 32A, 33, 31

**FIG. 13D** — 37, 32A, 33, 31

**FIG. 13E** — 32, 33, 31

**FIG. 13F** — 34, 32, 33, 31

**FIG. 13G** — 21, 36, 34, 32, 33, 31

**FIG. 13H** — 21, 36, 35, 34, 32, 33, 31, 20

FIG. 14

FIG. 15

**FIG. 16**

FIG. 17

# FIG. 18

EP 4 645 397 A1

**FIG. 19**

FIG. 20A

FIG. 20B

FIG. 20C

20(31)

62

20A

61

20(31)
62

62

20(31)

62

20(31)

**FIG. 20D**

20(31) 61

20A

62

20(31)

20(31)

62

**FIG. 20E**

20(31) 61

20A

62

20(31)

20(31)

20(31)

62

20(31)

20(31)

62

**FIG. 20F**

**FIG. 21**

**FIG. 22**

FIG. 23

# FIG. 24

FIG. 25A

FIG. 25B

FIG. 25C

FIG. 25D

FIG. 25E

FIG. 25F

## INTERNATIONAL SEARCH REPORT

| International application No. |
|---|
| **PCT/JP2023/041913** |

**A. CLASSIFICATION OF SUBJECT MATTER**

*H01L 33/62*(2010.01)i; *A01G 33/00*(2006.01)i; *C12M 1/00*(2006.01)i; *H01L 33/00*(2010.01)i
FI: H01L33/62; A01G33/00; C12M1/00 E; H01L33/00 L

According to International Patent Classification (IPC) or to both national classification and IPC

**B. FIELDS SEARCHED**

Minimum documentation searched (classification system followed by classification symbols)

H01L33/62; A01G33/00; C12M1/00; H01L33/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | KR 10-2019-0053720 A (SEOUL SEMICONDUCTOR CO., LTD.) 20 May 2019 (2019-05-20) paragraphs [0026]-[0138], fig. 1a-8d | 1-11, 13-14 |
| Y | paragraphs [0026]-[0138], fig. 1a-8d | 12, 15-17 |
| Y | WO 2012/002010 A1 (PANASONIC LIQUID CRYSTAL DISPLAY CO., LTD.) 05 January 2012 (2012-01-05) particularly, fig. 8-9 | 12 |
| Y | JP 2021-509754 A (HYUNDAI FOMEX CO., LTD.) 01 April 2021 (2021-04-01) particularly, paragraph [0029] | 15 |
| Y | JP 2013-38018 A (PANASONIC CORPORATION) 21 February 2013 (2013-02-21) particularly, fig. 5 | 16 |
| Y | JP 2019-180405 A (SCHOTT AG) 24 October 2019 (2019-10-24) paragraph [0025], fig. 1, 6-10 | 17 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 December 2023** | **26 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/041913**

| Patent document cited in search report | | | Publication date (day/month/year) | Patent family member(s) | | | Publication date (day/month/year) |
|---|---|---|---|---|---|---|---|
| KR | 10-2019-0053720 | A | 20 May 2019 | US | 2020/0219857 | A1 | |
| | | | | paragraphs [0041]-[0152], fig. 1A-8D | | | |
| | | | | DE | 202018006444 | U | |
| | | | | CN | 110678978 | A | |
| WO | 2012/002010 | A1 | 05 January 2012 | US | 2011/0316010 | A1 | |
| | | | | particularly, fig. 8-9 | | | |
| | | | | CN | 102971579 | A | |
| JP | 2021-509754 | A | 01 April 2021 | US | 2021/0020612 | A1 | |
| | | | | particularly, paragraph [0035] | | | |
| | | | | KR | 10-2009346 | B | |
| | | | | CN | 111051979 | A | |
| | | | | EP | 3636988 | A1 | |
| JP | 2013-38018 | A | 21 February 2013 | (Family: none) | | | |
| JP | 2019-180405 | A | 24 October 2019 | US | 2019/0309241 | A1 | |
| | | | | paragraph [0027], fig. 1, 6-10 | | | |
| | | | | EP | 3553161 | A1 | |
| | | | | DE | 102018108327 | A1 | |
| | | | | CN | 110358679 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012183002 A
- JP 2013251230 A

- JP 6006003 A